(19) Europäisches Patentamt
European Patent Office
Office européen des brevets

(11) EP 1 161 410 B1

(12) EUROPEAN PATENT SPECIFICATION

(45) Date of publication and mention of the grant of the patent:
**12.05.2004 Bulletin 2004/20**

(21) Application number: **99911748.4**

(22) Date of filing: **08.03.1999**

(51) Int Cl.[7]: **C07C 57/42**, C07C 59/64, C07C 47/232, A61K 31/19

(86) International application number:
**PCT/EP1999/001467**

(87) International publication number:
**WO 2000/053562 (14.09.2000 Gazette 2000/37)**

(54) **RETINOID ANTAGONISTS AND USE THEREOF**

RETINOID ANTAGONISTEN UND IHRE VERWENDUNG

ANTAGONISTES RETINOIQUES ET UTILISATION DE CEUX-CI

(84) Designated Contracting States:
**AT BE CH CY DE DK ES FI FR GB GR IE IT LI LU MC NL PT SE**

(43) Date of publication of application:
**12.12.2001 Bulletin 2001/50**

(73) Proprietor: **Basilea Pharmaceutica AG**
**4005 Basel (CH)**

(72) Inventors:
- **BOLLAG, Werner**
  **CH-4054 Basle (CH)**
- **KLAUS, Michael**
  **D-79576 Weil am Rhein (DE)**
- **MOHR, Peter**
  **CH-4054 Basle (CH)**
- **PANINA-BORDIGNON, Paola**
  **I-20129 Milan (IT)**
- **ROSENBERGER, Michael**
  **North Caldwell, NJ 07006 (US)**
- **SINIGAGLIA, Francesco**
  **I-20129 Milan (IT)**

(74) Representative: **Zimmermann, Hans, Dr. et al**
**c/o A. Braun Braun Héritier Eschmann AG**
**Patentanwälte VSP**
**Holbeinstrasse 36-38**
**Postfach 160**
**4003 Basel (CH)**

(56) References cited:
**WO-A-96/20913**
**WO-A-97/09297**
**US-A- 5 705 167**

- **S.S. CANAN KOCH ET AL: "Identification of the first retinoid X receptor homodimer antagonist" J. MED. CHEM., vol. 39, no. 17, - 1996 pages 3229-3234, XP002030143 cited in the application**

Remarks:
The file contains technical information submitted after the application was filed and not included in this specification

EP 1 161 410 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

## Description

Retinoids are a class of compounds structurally related to vitamin A, comprising natural and synthetic compounds. Retinoids have been found to be clinically useful in the treatment of dermatological and oncological diseases.

Retinoids with retinoid receptor agonistic activity have been shown to be active not only in model systems for the treatment of dermatological and oncological diseases but also in models for the treatment of T-helper cell type 1 (Th1)-mediated immune diseases. Retinoids with retinoid receptor agonistic activity are active in the treatment of adjuvant arthritis [Brinckerhoff et al., Science 221, 756-758 (1983)] and experimental allergic encephalomyelitis [Massacesi et al., J. Clin. Invest. 88, 1331-1337 (1991); Racke et al., J. Immunol. 154, 450-458 (1995)], animal models for rheumatoid arthritis and multiple sclerosis, respectively. Both diseases are considered to belong to Th1-mediated, immune diseases.

Experimentally, retinoids with retinoid receptor antagonistic activity (retinoid antagonists) are effective in counteracting many properties of retinoids with retinoid receptor agonistic activity (retinoid agonists) such as inhibition of cell proliferation, induction of cell differentiation, induction of apoptosis and inhibition of angiogenesis [Bollag et al., Int. J. Cancer 70, 470-472 (1997)]. Retinoid antagonists are also suppressing toxic side effects of retinoid agonists such as the signs and symptoms of the hypervitaminosis A syndrome and teratogenesis [Standeven et al., Toxicol. Appl. Pharmacol. 138, 169-175 (1996); Eckhardt and Schmitt, Toxicol. Letters 70, 299-308 (1994)]. Therefore, they may be useful clinically in preventing or treating adverse events caused by retinoid agonists.

Retinoid antagonists have been proposed for clinical use in prevention and therapy of retinoid-induced toxicity and side effects, particularly of the so-called hypervitaminosis A syndrome. Retinoid antagonists have also been proposed to be used in combination with retinoid receptor agonists or other nuclear receptor agonists for prevention and treatment of preneoplastic or neoplastic lesions, vitreo-retinopathy and retinal detachment. In addition, retinoid antagonists have been proposed for use as single agents, based on their anti-proliferative effect, for treatment of certain neoplasms insensitive to retinoid receptor agonists [WO 97/09297].

WO9620913A describes retinoids that show retinoid antagonist activity (compounds (I)) (see compounds 36, 38 and 40).

US-A-5705167 describes retinoid analogues of the general formula (I) that can present antagonistic activity (col. 5,1. 23-25) and can be used in various fields of therapy (col. 5, 1. 26-col. 6,1. 19).

WO 9709297A refers to the use of retinoid antagonists to provide a therapeutic effect against the pathological condition associated with retinoic acid receptor activity (p. 6, l. 12-16), being skin irritation and bone toxicity (p. 22, l. 4-18) explicitly acid (see examples 16, 17, 19, 21, 23 - 25, 60).

US-A-5391766 describes retinoic acid α-receptor (RARα) inhibitors.

In a first aspect, the present invention relates to novel retinoid antagonists. According to this particular aspect, the present invention relates to compounds of the formula I

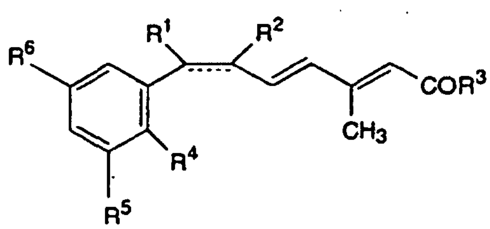

I

wherein the dotted bond is optional; and, when the dotted bond is present, $R^1$ is lower alkyl and $R^2$ is hydrogen; and, when the dotted bond is absent, $R^1$ and $R^2$ taken together are methylene to form a cis-substituted ryclopropyl ring; $R^3$ is hydroxy or lower alkoxy; $R^4$ is alkyl or alkoxy; and $R^5$ and $R^6$ are, independently, a $C_{4-12}$ alkyl group or a mono- or polycyclic $C_{5-12}$-hydrocarbon group which are linked to the phenyl ring through a quaternary carbon atom, and pharmaceutically acceptable salts of carboxylic acids of formula I.

As used herein the term "alkyl" means straight-chain, branched or cyclic alkyl residues, in particular those containing from 1 to 12 carbon atoms, such as methyl, ethyl, propyl, isopropyl, t-butyl, decyl, dodecyl, cyclopentyl, cyclohexyl, cycloheptyl and the like. The term "lower alkyl" means alkyl groups containing from 1 to 7, preferably 1-4 carbon atoms. Most preferred lower alkyl groups are methyl and ethyl. Alkyl and alkoxy groups denoted by $R^4$ preferably contain 1-8 carbon atoms, more preferably 1-4 carbon atoms. Particularly preferred group $R^4$ are ethoxy and butoxy. Examples of $C_{4-12}$ alkyl groups represented by $R^5$ or $R^6$ are tert.-butyl, 1,1-dimethylpropyl, 1-methyl-1-ethylpropyl, 1-methyl-1-ethylhexyl and 1,1-dimethyldecyl. Of these groups, tert.-butyl is preferred. Examples of mono- or polycyclic

hydrocarbon groups represented by $R^5$ and $R^6$ are 1-adamantyl and 1-methylcyclohexyl.

The compounds of formula I wherein $R^3$ is hydroxy form salts with pharmaceutically acceptable bases such as alkali salts, e.g. Na and K-salts, and ammonium or substituted ammonium salts such trimethylammonium salts which are within the scope of this invention.

In one embodiment, the invention comprises compounds of the formula I a

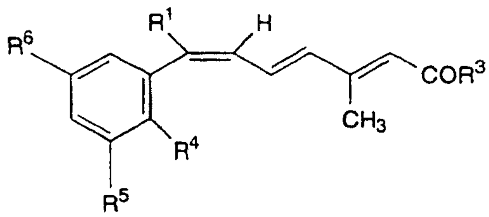

I a

wherein $R^1$ is lower alkyl and $R^3$ to $R^6$ are as in formula I;
and pharmaceutically acceptable salts of carboxylic acids of formula Ia.

In another embodiment the invention comprises compounds of the formula:

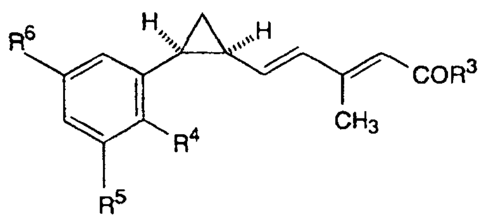

Ib

wherein $R^3$ to $R^6$ are as in formula I;
and pharmaceutically acceptable salts of carboxylic acids of formula Ib.

The compounds of formula I wherein $R^1$ and $R^2$ taken together are methylene, may be present in pure enantiomeric form or as racemates. While formula Ib arbitrarily depicts a particular enantiomeric form it is to be understood that the invention also comprises the opposite enantiomers as well as the racemates.

Particularly preferred are compounds of the formula Ia wherein $R^1$ is methyl, $R^4$ is ethoxy or butoxy and $R^5$ and $R^6$ are tert.-butyl.

The compounds of formula I above bind specifically to Retinoid X Receptors (RXR), but do not activate them. Accordingly the compounds of this invention can be used to reduce or abolish adverse events induced in patients with dermatological or oncological diseases. Experimental investigations on this subject are described in Examples 1-3.

In a second aspect, the present invention relates to the use of compounds of formula I for the manufacture of a medicament for the treatment of T-helper cell type 2 (Th2)-mediated immune diseases such as immunoglobulin E (IgE)-mediated allergic diseases, or diseases mediated by the Th2-related cytokines.

The term "retinoid antagonists" is used for retinoids or compounds with-RAR, RXR or mixed RAR-RXR antagonistic activity. It includes compounds with receptor neutral antagonistic activity (neutral antagonists), receptor inverse agonistic activity (inverse agonists) and negative hormone activity (negative hormones) [Klein et al., J. biol. Chem. 271, 22692-22696 (1996)].

Thus, the term "retinoid antagonists" encompasses

a) RXR antagonists of the formula I given earlier herein, particularly those of the formula

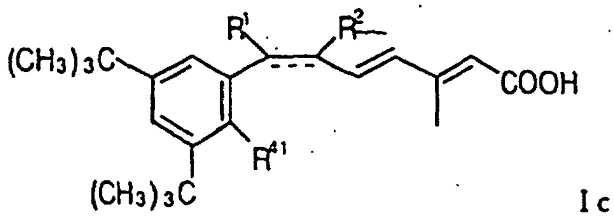

I c

wherein the dotted bond is optional; and, when the dotted bond is present, $R^1$ is methyl and $R^2$ is hydrogen; and, when the dotted bond is absent, $R^1$ and $R^2$ taken together are methylene to form a cis-substituted cydopropyl ring; and $R^{41}$ is $C_{1-4}$-alkoxy;

b) RAR $\alpha$-antagonists of formulae

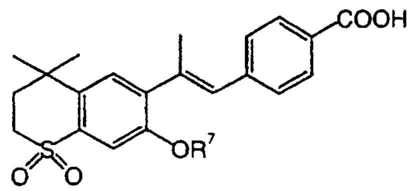

III

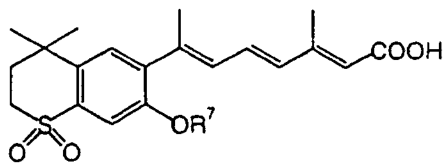

IV;

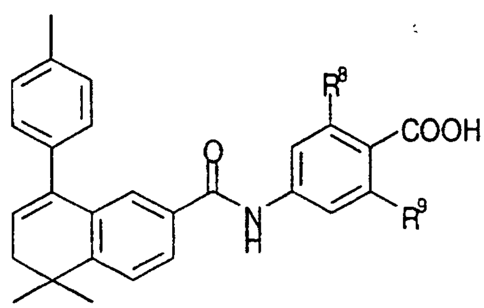

V;

and

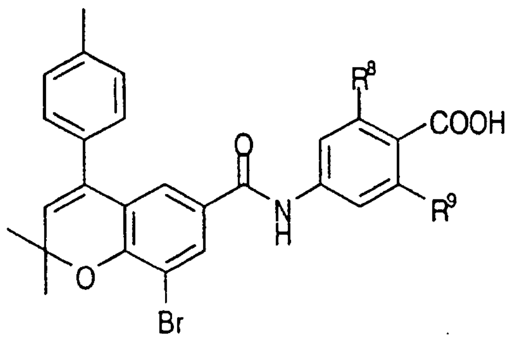

VI,

wherein $R^7$ is $C_{5-10}$-alkyl, and $R^8$ and $R^9$ independently of each other are hydrogen or fluorine; such compounds being described in US patent no. 5 391 766 and J. Med. Chem. 1997, 40, 2445;

c) RAR α,β antagonists of formulae

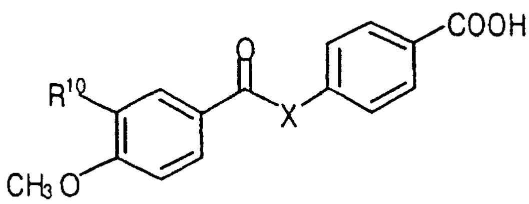

VII;

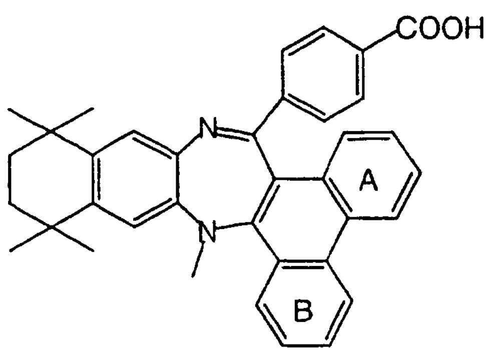

VIII;

and

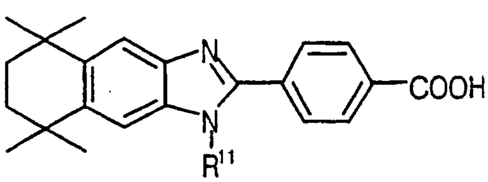

IX,

wherein $R^{10}$ is diamantyl, X is O or NH, $R^{11}$ is phenyl or benzyl, and wherein optionally either ring A or ring B is present;
such compounds being described in Med. Chem. Res. 1991, 1, 220; Biochem. Biophys. Res. Com. 1997, 231, 243; J. Med. Chem. 1994, 37, 1508;

d) RAR β,γ antagonists of formula

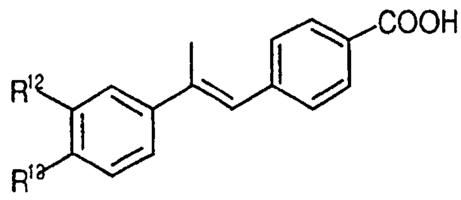

X,

wherein $R^{12}$ and $R^{13}$ independently of each other hydroxy, $C_{1-4}$-alkoxy, optionnaly branched $C_{1-5}$-alkyl or adamantyl;
such compounds being described in J. Med. Chem. 1995, 38, 4993;

e) RAR γ antagonists of formulae

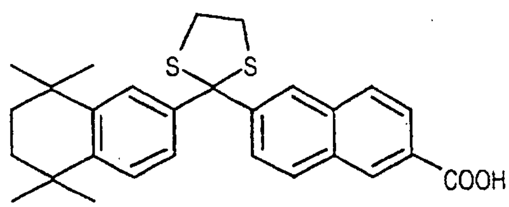

XI

and

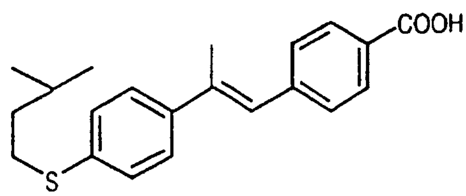

XII

such compounds being described in Cancer Res. 1995, 55, 4446;

f) RAR α,β,γ antagonists of formulae

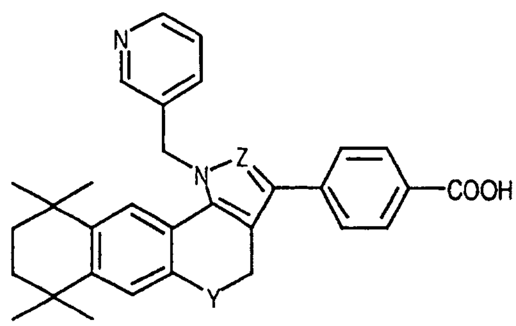

XIII;

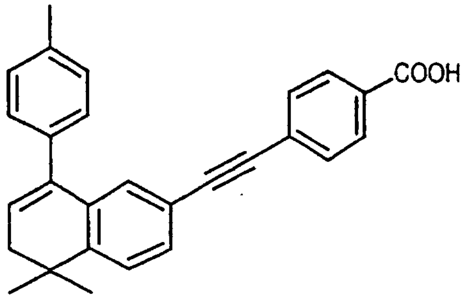

XIV,

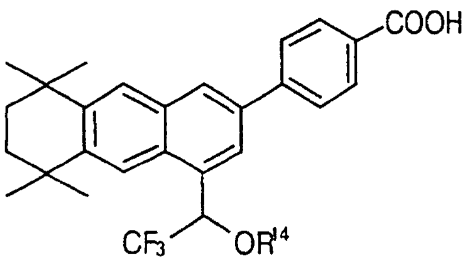

XV;

and

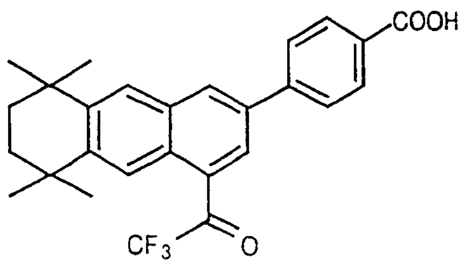

XVI

wherein Y is $-CH_2-$ or sulfur and Z is -CH= or nitrogen, and $R^{14}$ is hydrogen or $C_{1-4}$-alkyl; such compounds being described in J. Med. Chem. 1995, 38, 3163 and 4764; J. Biol. Chem. 1996, 271, 11897 and 22692;

g) RXR antagonists of formula

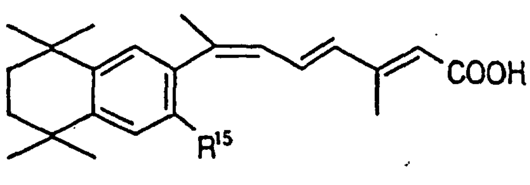

XVII;

wherein $R^{15}$ is $C_{1-4}$-alkoxy; such compounds being described in J. Med. Chem. 1996, 39, 3229; and Nature 1996, 383, 450,

as well as pharmaceutically acceptable salts and pharmaceutically acceptable hydrolyzable esters of the compounds of formulae III to XVII.

In the scope of the present invention, the "pharmaceutically acceptable salts" includes any salt chemically

permissible in the art for retinoid antagonists and applicable to human patients in a pharmaceutically acceptable preparation. Any such conventional pharmaceutically acceptable salt of retinoid antagonists can be utilized. Among the conventional salts which can be utilized, there are the base salts included, for example, alkali metal salts such as the sodium or potassium salt, alkaline earth metal salts such as the calcium or magnesium salt, and ammonium or alkyl ammonium salts.

In accordance with this second aspect of the invention, it has thus been found that administration of compounds of formula I, pharmaceutically acceptable salts, and pharmaceutically acceptable hydrolyzable esters thereof, are efficacious in treating patients with T-helper cell type 2 (Th2)-mediated diseases. It has also been found that the administration of compounds of formula I is efficacious in treating patients with diseases mediated by Th2-related cytokines, such as interleukin-4 (IL-4) and IL-5.

This aspect of the invention, therefore, in one embodiment relates to the use of compounds of formula I, their pharmaceutically acceptable salts or pharmaceutically acceptable hydrolyzable esters, for the manufacture of a medicament for the treatment of T-helper cell type 2 (Th2)-mediated immune diseases. In another embodiment this aspect of the invention relates to the use of compounds of formula I, their pharmaceutically acceptable salts or pharmaceutically acceptable esters thereof for the manufacture of a medicament for the treatment of disease mediated by Th2-related cytokines, such as IL-4 and IL-5. In this respect the invention enables a method for treating patients having T-helper cell type 2 (Th2)- mediated immune diseases comprising administering to said human patient a compound selected from the group of compounds of formula I, pharmaceutically acceptable salts and pharmaceutically acceptable hydrolyzable esters thereof, said compound being administered in an amount effective to treat said disease. The term "treatment" or "treating" includes preventive and/or therapeutic treatment.

As used herein, the term "T-helper cell type 2-mediated immune diseases" relates to diseases involving immunoglobulin E (IgE) and mast cells due to the development and activation of allergen-specific Th2 cells and it encompasses allergic diseases, such as atopic dermatitis, other dermatological diseases associated with atopy; allergic rhinitis or hay fever, allergic bronchial asthma in its acute or chronic, mild or severe forms, with or without acute or chronic bronchitis. Elevated serum levels of immunoglobulin E (IgE) and hypereosinophilia can be associated with these diseases. Retinoid antagonists are effective in all those immune diseases which are linked with an increase of Th2 cell activity and an increased secretion of the related cytokines, such as IL-4 and IL-5. The therapeutic effect of retinoid antagonists is supposedly due to a decrease in Th2 cell activity, a decreased secretion of the related cytokines, such as IL-4 and IL-5, and/or an increase in Th1 cell activity due to the enhancement of IL-12 production by activated myelomonocytic cells. [S. Romagnani, Ann. Rev. Immunol. 12,227-257 (1994); Romagnani, ed., Th1 and Th2 Cells in Health and Disease. Chem. Immunol., Karger, Basel, 63, pp. 187-203 (1996); Abbas et al., Nature 383, 787-793 (1996)].

The efficacy of the retinoid antagonists in accordance with the present invention can be shown by their ability to either upregulate Th1 cell activity or induce/stimulate the production of cytokines, such as IL-12, IFNγ, TNF; and/or down-regulate Th2 cell activity, or inhibit the production of cytokines, such as IL-4 and IL-5 (see Examples 4 and 5 below).

Retinoid antagonists are active in the treatment of allergic bronchial asthma. The hallmarks of inflammation associated with asthmatic disease are the presence of activated eosinophils, an increased sensitivity of the airways (hyperresponsiveness), edema, mucus hypersecretion and cough. This inflammatory process is mediated by the generation and activation of Th2-type cells. The ability of retinoid antagonists to promote a Th1-type response and thereby to suppress the Th2-type response is thought to be the mechanism underlying the efficacy of these compounds in allergic lung inflammation/asthma. Retinoid antagonists are acting on Th1-type cells, in inhibiting the signs and symptoms of allergic lung inflammation/asthma [Gavett et al., J. exp. Med. 182, 1527-1536 (1995); Kips et al., Am. J. Respir. Crit. Care Med. 153, 535-539 (1996)]. They are active in antigen/allergen (e.g. ovalbumin)-sensitized and challenged animals. Retinoid antagonists, given either systemically or topically by aerosol, are efficacious in attenuating, inhibiting or reversing bronchoconstriction, airway edema and mucus hypersecretion, airway inflammation, accumulation of eosinophils and neutrophils in the broncho-alveolar tissue and broncho-alveolar lavage respectively, as well as airway hyperresponsiveness to non-specific stimuli (see Example 6 below).

For the treatment, the active compound, i.e. a retinoid antagonist, a pharmaceutically acceptable salt or a pharmaceutically acceptable hydrolyzable ester thereof, is administered either systemically or topically. Preferably, said compound is administered as a composition containing said active compound and a pharmaceutically acceptable carrier or diluent compatible with said active compound. In preparing such composition, any conventional pharmaceutically acceptable carrier can be utilized. When the drug is administered orally, it is generally administered at regular intervals, conveniently at mealtimes or once daily. It has been established that this compound is effective in doses which show no or only mild side effects when given orally or when given topically. Therefore, oral or topical administration of the active compound is generally preferred. For treating diseases of the skin, mouth, nose, pharynx, larynx, bronchus etc. oral combined with topical administration may also be used advantageously.

In a third aspect of this invention, it has been found that administration of compounds of formula I, pharmaceutically acceptable salts, and pharmaceutically acceptable hydrolyzable esters thereof, are efficacious in treating

patients with osteoporosis.

The invention, therefore, in that aspect, relates to the use of compounds of formula I, their pharmaceutically acceptable salts or pharmaceutically acceptable hydrolyzable esters, for the manufacture of a medicament for the treatment of osteoporosis.

Accordingly, the invention also enables a method for treating patients having osteoporosis comprising administering to said human patient a compound selected from the group of compounds of formula I, pharmaceutically acceptable salts and pharmaceutically acceptable hydrolyzable esters thereof, said compound being administered in an amount effective to treat said disease. The term "treatment" or "treating" includes preventive and/or therapeutic treatment.

As used herein the term "osteoporosis" includes primary as well as secondary osteoporosis and relates to a disease characterized by a low bone mass and a micro-architectural deterioriation of bone tissue leading to enhanced bone fragility and a consequent increase in bone fracture risk.

The two most common forms of primary osteoporosis are: 1. The postmenopausal osteoporosis in women (osteoporosis type I) with a high rate of bone remodeling, also called high turnover form of osteoporosis. The increased resorption of trabecular bone leads most often to vertebral and wrist fractures. 2. Senile osteoporosis in individuals of both sexes mostly over 70 years (osteoporosis type II) with a low rate of bone remodeling called low turnover form of osteoporosis. The increased resorption of both trabecular and cortical bone leads frequently to fractures of vertebrae and femoral neck.

Secondary osteoporosis is related to many conditions such as diseases (e.g. rheumatoid arthritis, tumor-induced osteolysis with or without hypercalcemia, intestinal and renal disorders), immobilisation/lack of exercise, malnutrition, disorders of calcium intake or vitamin D intake and their metabolism, disorders of parathyroid hormone metabolism, and drug therapy (e.g. corticosteroids, heparin).

The efficacy of retinoid antagonists in accordance with the present invention can be shown by their ability to influence bone remodeling, a process resulting from bone resorption and bone formation. Bone resorption is mainly accomplished by multinucleated osteoclasts endowed with specialized organelles and plasma membrane structures to allow resorption of inorganic substances as well as organic matrix components. Bone formation is mainly a function of osteoblasts that build up the extracellular matrix consisting of proteoglycans, type I collagen, non-collagen proteins, osteonectin, osteocalcin and other components. A further function of osteoblasts is bone mineralization, involving induction of certain enzymes (e.g. alkaline phosphatase), incorporation of calcium and other components into the inorganic bone structure, such as hydroxyapatite.

When bone resorption rate is higher than bone formation, it leads to a net reduction in bone mass, which occurs in osteoporosis. Retinoid antagonists decrease bone resorption and/or increase bone formation and are therefore useful in prevention and therapy of osteoporosis.

Under physiological and pathological conditions a series of compounds are known to influence and particularly induce or stimulate bone resorption such as hormones, vitamines, growth factors, cytokines, prostaglandins, lipopolysaccharide etc. (Reviews are given in the book Principles of Bone Biology, Eds. J.B. Bilezikian et al., Academic Press, San Diego 1996: Horowitz MC et al., Local regulators of bone, pp. 687-700. Pilbeam CC et al., Prostaglandins and bone metabolism, pp. 715-728. Mundy GR, Role of cytokines, parathyroid hormone and growth factors in malignancy, pp. 827-836. Rodan GA et al., Pathophysiology of osteoporosis, pp. 979-990. Jones G, Pharmacological mechanism of therapeutics: Vitamin D and analogs, pp. 1069-1081. Hakeda et al., The growth and culture of bone cells: Osteoclastic, pp. 1217-1228. Geddes AD, Animal models of bone diseases, pp. 1343-1354).

The following compounds known as inducing/stimulating bone resorption were examined: Parathyroid hormone (PTH), parathyroid hormone related peptide (PTHrP), calcitriol (1,25-dihydroxyvitamin $D_3$), all-trans retinoic acid (all-trans RA), 9-cis retinoic acid (9-cis RA), prostaglandin E 2(PGE2), tumor necrosis factor $\alpha$ (TNF$\alpha$), and interleukin-1$\alpha$ (IL-1$\alpha$); Vaes G, Cellular biology and biological mechanism of bone resorption, Clinical Orthopaedics and related Research, 1988, 231, 239-271. Houghs S. et al., Effects of hypervitaminosis A on the bone and mineral metabolism of the rat, Endocrinology 1988, 122, 2933-2939. Tullberg-Reinert H. et al., Different inhibitory actions of cyclosporin A and cyclosporin A-acetate on lipopolysaccharide-, interleukin l-, 1,25-dihydroxyvitamin $D_3$-, and parathyroid hormone-stimulated calcium and lysosomal enzyme release from mouse calvaria in vitro. Agents and Actions 1991, 32, 321-332. Ammann P. et al., Effects of the biphosphonate tiludronate on bone resorption, calcium balance and bone mineral density. J. Bone Miner. Res. 1993, 8, 1491-1498. Bonjour JP et al., Tiludronate: Bone pharmacology and safety. Bone 1995, 17, 473S-477S. Kindmark A et al., Inhibitory effects of 9-cis and all-trans retinoic acid on 1,25$(OH)_2$ vitamin $D_3$-induced bone resorption Calcif. Tissue Int. 1995, 57, 242-244. Saneshige S., Retinoic acid directly stimulates osteoclastic bone resorption and gene expression of cathepsinK/OC-2. Biochem. J. 1995, 309, 721-724.

It could be demonstrated that retinoid antagonists inhibit bone resorption induced by above mentioned resorption stimulating compounds. This was proven in the widely used model of neonatal mouse calvaria (skull bones) tissue culture, a model predictive for agents useful in clinical prevention and therapy of osteoporosis e.g. biphosphonates. The experiments are described below in Example 7.

In a fourth aspect the present invention relates to the use of compounds of formula in the manufacture of a medicament for the treatment of preneoplastic and neoplastic diseases such as precancerous lesions of the skin and mucous membranes, or solid tumors of skin, mucous membranes, head and neck, lung, stomach, colon, breast, ovary, cervix and prostate; and (see Example 8 below).

For the treatment of the above-mentioned diseases, the active compound, i.e. a retinoid antagonist, a pharmaceutically acceptable salt or a pharmaceutically acceptable hydrolyzable ester thereof, is administered either systemically or topically. Preferably, said compound is administered as a composition containing said active compound and a pharmaceutically acceptable carrier or diluent compatible with said active compound. In preparing such composition, any conventional pharmaceutically acceptable carrier can be utilized. When the drug is administered orally, it is generally administered at regular intervals, conveniently at mealtimes or once daily. It has been established that this compound is effective in doses which show no or only mild side effects when given orally or when given topically. Therefore, oral or topical administration of the active compound is generally preferred. For treating diseases of the skin, mouth, nose, pharynx, larynx, bronchus etc. oral combined with topical administration may also be used advantageously.

In the treatment of the above-mentioned diseases, retinoid antagonists, when administered orally do not or only slightly induce the adverse events belonging to the toxic syndrome of hypervitaminosis A, such as mucocutaneous, musculoskeletal, neurologic manifestations and elevation of transaminases, triglycerides and cholesterol. In addition, they are not or less teratogenic in contrast to the receptor agonistic retinoids clinically useful in the treatment of dermatological and oncological diseases, such as all-trans retinoic acid (tretinoin), 13-cis retinoic acid (isotretinoin), etretinate and acitretin.

In the treatment of T-helper cell type 2-mediated immune diseases, retinoid antagonists, pharmaceutically acceptable salts or pharmaceutically acceptable hydrolyzable esters thereof, can be used alone or in combination with other measures, e.g. in combination with other pharmaceutically active substances such as topical or systemic corticosteroids, antihistaminics and bronchodilating agents. If used in combination with other substances, retinoid antagonists and said other substances can be administered separately or incorporated in effective amounts into one pharmaceutical composition.

In the treatment of osteoporosis, retinoid antagonists, pharmaceutically acceptable salts or pharmaceutically acceptable hydrolyzable esters thereof, can be used alone or in combination with other measures, e.g. in combination with other pharmaceutically active substances such as calcium, vitamin D derivatives, estrogens, anabolics, calcitonin or biphosphonates. If used in combination with other substances, retinoid antagonists and said other substances can be administered separately or incorporated in effective amounts into one pharmaceutical composition.

The retinoid antagonists can also be administered in the form of its pharmaceutically acceptable hydrolyzable esters. Any pharmaceutically acceptable hydrolyzable ester can be used in the compositions and methods of this invention. Among the preferred esters are: the aromatic esters such as benzyl esters in which the benzyl moiety is unsubstituted or substituted with lower alkyl, halo, nitro, thio, or substituted thio; or lower alkyl esters, e.g. ethyl, t-butyl, cyclopentyl, cyclohexyl or cycloheptyl ester; or 9-fluorenylmethyl ester.

The aforementioned retinoid antagonists, the salts and esters thereof are useful especially in pharmaceutically acceptable oral or topical modes. These pharmaceutical compositions contain said active compound in association with a compatible pharmaceutically acceptable carrier material. Any conventional carrier material can be utilized. The carrier material can be organic or inorganic inert carrier material suitable for oral administration. Suitable carriers include water, gelatin, gum arabic, lactose, starch, magnesium stearate, talc, vegetable oils, polyalkylene-glycols, petroleum jelly and the like. Furthermore, the pharmaceutically active preparations may contain other pharmaceutically active agents. Additionally, additives such as flavouring agents, preservatives, stabilizers, emulsifying agents, buffers and the like may be added in accordance with accepted practices of pharmaceutical compounding.

The pharmaceutical preparations can be made up in any conventional form including inter alia: (a) a solid form for oral administration such as tablets, capsules (e.g. hard or soft gelatine capsules), pills, sachets, powders, granules, and the like; (b) preparations for topical administrations such as solutions, suspensions, ointments, creams, gels, micronized powders, sprays, aerosols and the like. The pharmaceutical preparations may be sterilized and/or may contain adjuvants such as preservatives, stabilizers, wetting agents, emulsifiers, salts for varying the osmotic pressure and/or buffers.

For topical administration to the skin or mucous membrane the aforementioned derivative is preferably prepared as ointments, tinctures, creams, gels, solution, lotions, sprays; aerosols and dry powder for inhalation, suspensions, shampoos, hair soaps, perfumes and the like. In fact, any conventional composition can be utilized in this invention. Among the preferred methods of applying the composition containing the agents of this invention is in the form of an ointment, gel, cream, lotion, spray; aerosol or dry powder for inhalation. The pharmaceutical preparation for topical administration to the skin can be prepared by mixing the aforementioned active ingredient with non-toxic, therapeutically inert, solid or liquid carriers customarily used in such preparation. These preparations generally contain 0.01 to 5.0 percent by weight, preferably 0.1 to 1.0 percent by weight, of the active ingredient, based on the total weight

of the composition.

In preparing the topical preparations described above, additives such as preservatives, thickeners, perfumes and the like conventional in the art of pharmaceutical compounding of topical preparation can be used. In addition, conventional antioxidants or mixtures of conventional antioxidants can be incorporated into the topical preparations containing the aforementioned active agent. Among the conventional antioxidants which can be utilized in these preparations are included N-methyl-α-tocopherolamine, tocopherols, butylated hydroxyanisole, butylated hydroxytoluene, ethoxyquin and the like. Cream-base pharmaceutical formulations containing the active agent, used in accordance with this invention, are composed of aqueous emulsions containing a fatty acid alcohol, semi-solid petroleum hydrocarbon, ethylene glycol and an emulsifying agent.

Ointment formulations containing the active agent in accordance with this invention comprise admixtures of a semi-solid petroleum hydrocarbon with a solvent dispersion of the active material. Cream compositions containing the active ingredient for use in this invention preferably comprise emulsions formed from a water phase of a humectant, a viscosity stabilizer and water, an oil phase of a fatty acid alcohol, a semi-solid petroleum hydrocarbon and an emulsifying agent and a phase containing the active agent dispersed in an aqueous stabilizer-buffer solution. Stabilizers may be added to the topical preparation. Any conventional stabilizer can be utilized in accordance with this invention. In the oil phase, fatty acid alcohol components function as a stabilizer. These fatty acid alcohol components function as a stabilizer. These fatty acid alcohol components are derived from the reduction of a long-chain saturated fatty acid containing at least 14 carbon atoms. Also, conventional perfumes and lotions generally utilized in topical preparation for the hair can be utilized in accordance with this invention. Furthermore, if desired, conventional emulsifying agents can be utilized in the topical preparations of this invention.

For topical treatment of allergic rhinitis and allergic bronchial asthma nasal and inhalation aerosols are used. Formulations for such aerosols are described in Drugs and Pharmaceutical Sciences, Marcel Dekker, New York, 1996, Vol. 72, pp. 547-574. Furthermore, the active compound can be delivered by dry powder inhalation. Such formulations and devices are described in Pharmaceutical Technology, June 1997, pp. 117-125.

A preferred oral dosage form comprises tablets, pills, sachets, or capsules of hard or soft gelatin, methylcellulose or of another suitable material easily dissolved in the digestive tract. Each tablet, pill, sachet or capsule can preferably contain from about 5 to about 200 mg, more preferably from about 20 to about 100 mg, of active ingredient. The oral dosages contemplated in accordance with the present invention will vary in accordance with the needs of the individual patient as determined by the prescribing physician. Generally, however, a daily dosage of from 0.05 to 20 mg per kg of body weight, preferably 0.1 to 7 mg, and most preferably from about 0.3 mg to about 1.5 mg per kg of body weight of the patient is utilized. This dosage may be administered according to any dosage schedule determined by the physician in accordance with the requirements of the patient.

The dosage for treatment typically depends on the route of administration, the age, weight and disease condition of the individual. Suitable dosage forms are known in the art or can be easily obtained in a manner known per se. Formulations of lotions, gels, creams, sprays; aerosols and dry powder for inhalation, hard or soft gelatin capsules, tablets and sachets that are particularly suitable in the scope of the present invention or that can be easily adjusted in accordance with the above teaching are in the art.

The pharmacological activity of the retinoid antagonists as disclosed above can be demonstrated in various test models as shown below in Examples 1-8. In Examples 1-8 the following compounds were used:

| | |
|---|---|
| Compound A: | (2E,4E,6Z)-7-(3,5-di-tert.butyl-2-ethaxyphenyl]-3-methyl-2,4,6-octatrienoic acid |
| Compound B: | (2E,4E,6Z)-7-(3,5-di-tert.butyl-2-butyloxyphenyl]-3-methyl-2,4,6-octatrienoic acid |
| Compound C: | all-trans retinoic acid |
| Compound D: | 13-cis retinoic acid |
| Compound E: | 9-cis retinoic acid |
| Compound F: | 4-(5,6,7,8-tetrahydro-5,5,8,8-tetramethyl-2-naphthalene-carboxamido)benzoic acid |
| Compound G: | (2E,4E)-3-methyl-5-[(1S,2S)-2-(5,5,8,8-tetramethyl-5,6,7,8-tetrahydronaphthalen-2-yl)-cyclopropyl]-penta-2,4-dienoic acid |
| Compound H: | p-[(E)-2-[3',4'-Dihydro-4',4'-dimethyl-7'-(beptyloxy)-2'H-1-benzothiopyran-6'-yl]propenyl]benzoic acid 1',1'-dioxide |
| Compound i: | 4-(7,7,10,10-Tetramethyl-1-pyridin-3-ylmethyl-4,5,7,8,9,10-hexahydro-1H-naphto[2,3-g]indol-3-yl)-benzoic acid |

Example 1

Effect of retinoids with RXR antagonistic activity on weight loss induced by all-trans retinoic acid in mice.

The test compounds were solubilized/suspended in arachis oil and applied intraperitoneally into female mice

(25/30 g) for 5 days a week during 2 weeks. The results are shown in Table 1.

Table 1

| Compound/Dose [mg/kg] | C/200 | C/200 + A/200 | C/100 | C/100 + A/200 | A/200 |
|---|---|---|---|---|---|
| | n=2 | n=2 | n=10 | n=2 | n=2 |
| % survival after 14 days | 0 | 100 | 20 | 100 | 100 |
| % weight change after 4 days | -38 | -23 | -18 | -13 | +15 |
| % weight change after 7 days | dead | -23 | -29 | -8 | +26 |

Example 2

Suppressive effect on skin irritation caused by all-trans retinoic acid in hairless rats.

The animals were treated epicutaneously (0.025 ml/2 $cm^2$) once a day, 5 days a week (Monday to Friday) for 4 consecutive weeks with the mixed solution of the test compounds in acetone/ethanol (1/1).

The animals were observed daily, Monday to Friday, for signs of erythema and oedema on each individual test site, starting at approximately 24 hours after initiating the study. The intensity of all skin reactions induced was recorded in the raw data sheets according to the following grading scale:

0 = No skin reactions
1 = Slight skin reactions (slight to defined erythema)
2 = Well defined reaction (well defined to marked erythema)
3 = Moderate skin reaction (moderate to marked erythema with defined oedema)
4 = Severe skin reaction (severe to strong erythema and marked oedema)

The mean cumulative skin irritation scores calculated after each treatment application are presented in Figures 1 and 2.

Example 3

Suppressive effect on the in vitro teratogenic activity of various retinoids.

Fore- and hindlimb buds of day 13 rat embryos were dissociated in calcium-magnesium-free balanced salt solution containing 0.1% trypsin and 0.1% EDTA at 37°C. The cell density was adjusted to $2x10^7$ limb bud cells/ml in CMRL medium containing 10% NU-serum. High cell density cultures were set up by dispensing 20µl of the cell suspension as a discrete drop in the center of each well of a 24-well dish. The cells were allowed to attach for 1-2 h at 37°C before the cultures were flooded with the appropriate culture medium. The test substances then were dissolved in dimethylsulfoxide and added at the same time on day 1 of culture. An equivalent amount of the solvent (0.4%) was added to the control cultures. After 7 days in culture the grade of differentiation of mesenchymal cells into chondrocytes, producing cartilage was determined by staining proteoglycans with alcian blue. The bound dye was extracted with 4 M guanidine hydro-chloride and the absorbance at 600 nm was determined spectrophotometrically.

Figure 3 shows the interaction of variable concentrations of Compound A with a constant concentration of all-trans retinoic acid (Compound C). C was kept during the experiment at a constant concentration of $1x10^{-6}$ mol/l. The concentrations of Compound A increased from $1x10^{-9}$ mol/l to $2x10^{-6}$ mol/l. The differentiation of the mesenchymal cells increased from about 5% to about 30% with increasing concentrations of Compound A.

Figure 4 shows the interaction of variable concentrations of Compound A ($1x10^{-9}$ mol/l to $2x10^{-6}$ mol/l) with Compound D which was kept during incubation at the constant concentration of $1x10^{-6}$ mol/l. The differentiation of the mesenchymal cells increased from about 30% to about 70% with increasing concentrations of Compound A.

Figure 5 shows the interaction of variable concentrations of Compound A ($1x10^{-9}$ mol/l up to $2x10^{-6}$ mol/l) with Compound E, which was kept during incubation at the constant concentration of $5x10^{-7}$ mol/l. The differentiation of the mesenchymal cells increased from about 5% to about 80% with increasing concentrations of Compound A.

Figure 6 shows the interaction of variable concentrations of Compound A ($1x10^{-9}$ mol/l up to $2x10^{-6}$ mol/l) with Compound F which was kept during incubation at the constant concentration of $1x10^{-8}$ mol/l. The differentiation of the mesenchymal cells increased from about 5% to about 75% with increasing concentrations of Compound A.

Figure 7 shows the interaction of variable concentrations of Compound A ($1x10^{-9}$ mol/l up to $2x10^{-6}$ mol/l)

with Compound G, which was kept during incubation at the constant concentration of $1x10^{-6}$ mol/l. The differentiation of the mesenchymal cells increased from about 5% to about 110% with increasing concentrations of Compound A.

Example 4

*In vitro* assay for induction of IL-12 production by retinoid antagonists

THP-1 cells were obtained from American Tissue Culture Collection and cultured in complete medium. To assay for IL-12 production, THP-1 cells, 1.25 x $10^6$ cells/ml, were stimulated with *S. aureus* Cowan strain (SAC) (1/1000) and human recombinant interferon-γ (huIFN-γ (1000 U/ml) [Ma et al., *J. Exp. Med*. 183, 147-157 (1996)]. Alternatively, 0.5 x $10^6$ human peripheral blood mononuclear cells (PBMC) (1 ml culture in 48 well plates) were primed with huIFN-γ (1000 U/ml) for 16 hours at 37°C, and then stimulated with SAC (1/1000). Supernatants were collected after 48 hours and freezed at -20 °C until assayed [Panina-Bordignon et al., *J. Clin. Invest*. 100, 1513-1519 (1997)].

IL-12 production was measured by specific enzyme linked immuno sorbant assay (ELISA), using 20C2 antibody (rat anti human IL-12 heterodimer p40-p35), at 2.5 μg/ml in coating buffer, and peroxidase-conjugated 4D6 antibody (rat anti human IL-12) at 250 ng/ml in assay buffer as described [Zhang et al., *J. Clin. Invest*. 93, 1733-1739 (1994)]. Standard (recombinant human IL-12, 800 pg/ml to 6 pg/ml) and samples (100 μl) diluted in assay buffer were added to duplicate wells. Absorbance was read at 450-650 nm. The unknown IL-12 concentrations of the samples were read from the corresponding standard curve and multiplied by the corresponding dilution factor. Maximal IL-12 production varied between 200 and 400 pg/ml.

Lyophilized retinoid antagonists were diluted in DMSO under yellow light, on ice at a concentration of 2 mM. Serial dilutions (1 μM-1 pM) were prepared in complete RPMI medium. 10 μl of each dilution was added to 1 ml culture.

The results of the experiments indicate that the tested retinoid antagonists influence IL-12 production. In particular, the tested retinoid antagonists stimulate IL-12 production by activated human monocytes, see Table 2 and 3

Table 2

**Retinoid antagonists specifically enhance IL-12 production by activated monocytes**

| | nM | IL-12 (pg/ml) | IL-10 (pg/ml) | TNF-α (pg/ml) |
|---|---|---|---|---|
| medium | | 0 | <10 | <10 |
| SAC+IFN-γ | | 120 | 1040 | 1840 |
| RAR α antagonist | 1000 | 251 | 1343 | 1912 |
| Compound H | 100 | 102 | 1050 | 1600 |
| | 10 | n.d. | 1060 | 1392 |
| medium | | 0 | <10 | <10 |
| SAC+IFN-γ | | 126 | 1040 | 2000 |
| RAR α,β,γ antagonist | 1000 | 321 | 1116 | 2884 |
| Compound I | 100 | 205 | 983 | 2752 |
| | 10 | 173 | 971 | 2592 |
| medium | | 0 | <10 | <10 |
| SAC+IFN-γ | | 120 | 1040 | 1840 |
| RXR antagonist | 1000 | 298 | 1700 | 1560 |
| Compound B | 100 | 161 | 1521 | 1812 |
| | 10 | 106 | 1020 | 1484 |

Table 3

| Retinoid antagonists enhance IL-12 production by PBMC and THP-1 cells that have been primed with IFNγ and stimulated with SAC | | | | | | |
|---|---|---|---|---|---|---|
| Compound | Receptor Specificity | Activity | Stimuli | Time * (hrs) | PBMC IL-12 | THP-1 (pg/ml) |
| H | RAR α | antagonist | IFNγ+<br>SAC | 0<br>16 | 503<br>401 | 306<br>nd |
| I | RAR α,β,γ | antagonist | IFNγ+<br>SAC | 0<br>16 | 371<br>367 | 364<br>nd |
| B | RXR | antagonist | IFNγ+<br>SAC | 0<br>16 | 568<br>367 | 577<br>nd |
| none | | | none<br>IFNγ+ SAC | | <12<br>360 | <2<br>275 |

* retinoid antagonists (1 μg) were added at time 0 together with IFNγ or after 16 hours together with SAC.

Example 5

In vitro assay for inhibition of differentiation of human naive T cells into T helper 2 (Th2) cells by retinoid antagonists.

Naive T cells from cord blood were isolated and treated as described [Panina-Bordignon et al. *J. Clin. Invest.* 100. 1513-1519 (1997)]. Briefly, cord blood derived mononuclear cells were incubated with anti-CD45RA and anti-CD4 monoclonal antibodies. After a 20 minute incubation, cells were washed and incubated with goat antimouse Ig-coated magnetic beads. Positive cells were separated and seeded at $1 \times 10^6$ cells/ml in a 24 well plate, together with autologous adherent cells, PHA, and IL-4 in the presence or absence of Compound H or Compound B at 1mM for 5 days. Cells were then washed and put back in culture in the presence of IL-2 (100 U/ml). After 10 days, the cells were collected and restimulated with PMA (50 ng/ml) and ionomycin (1 μg/ml) for 4 hours. Brefeldin A (10 μg/ml) was added for the last 2 hours. Then the cells were fixed with 4% paraformaldehyde and permeabilized with saponin. Fixed cells were stained with FITC-anti IFNγ and PE-anti-IL-4mAbs and subjected to cytofluorimetric analysis.

The results of the experiment indicate that the tested retinoid antagonists reduce the differentiation of naive T cells into IL-4-secreting Th2 cells. (Table 4)

Table 4

| Suppression of IL-4 expression in Th2 cells by retinoid antagonists | | |
|---|---|---|
| | IL-4 expressing cells | |
| | % gated cells | % Th2 cells |
| Th2 | 26.32 | 100 |
| Th2 + Compound H | 10.8 | 41 |
| Th2 + Compound B | 8.5 | 32 |

Example 6

Murine model of allergen-induced airway inflammation and hyperresponsiveness.

C57BL/6 mice (8-9 weeks old) are actively sensitized to ovalbumin (OA) on day 0 and on day 14 by a intraperitoneal injection of 10 μg OA + 1 mg $Al(OH)_3$ (gel suspension) in 0.2 ml sterile saline. On day 21, the mice were challenged with 5.0 % OA aerosol for 18 minutes. The aerosol is generated by a De Vilbiss Ultra-Neb 90 ultrasonic nebulizer, the outlet of which is connected to a small plexiglass chamber containing the animals. The mice are dosed with the RXR antagonist Compound B (10 and 30 mg/kg intraperitoneally) daily for three days, 48 hours, 24 hours, and immediately prior to OA challenge. Animals are used on day 21.

Airway Inflammatory Cell Accumulation: On day 24, three days after the challenge with OA aerosol, animals

are anesthetized with urethane (2.4 g/kg) and tracheotomized with a 23 gauge catheter. Lungs are lavaged with aliquots (2 x 1 ml) of sterile Hank's balanced salt solution without $Ca^{++}$ and $Mg^{++}$. Lavage fluid is recovered after 30 sec by gentle aspiration and pooled for each animal. Samples then are centrifuged at 2000 rpm for 15 minutes at 5 °C. Red blood cells are lysed from the resulting pellet with 0.5 ml distilled water and the cells remaining in the pellet are reconstituted with 5 ml HBSS. Samples are centrifuged a second time at 2000 rpm for 15 minutes at 5 °C. The resulting pellet is suspended in 1 ml of HBSS. Total cell number is determined from an aliquot of the cell suspension using a hemocytometer. For cytological preparations, the cells are fixed on cytocentrifuged slides stained with a modified Wright's stain. Differential counts on at least 300 cells are made using standard morphological criteria to classify cells.

The results of the experiments indicate that the tested retinoid antagonists inhibit the allergen-induced accumulation of airway inflammatory cells (Table 5)

Table 5

| Suppression of airway inflammatory cell accumulation by retinoid antagonists in a mouse model of allergen-induced airway inflammation | | | | | |
|---|---|---|---|---|---|
| | Cell Influx (cells/ml) | | | Percent of reduction | |
| | Vehicle | Compound B | | Compound B | |
| | | 10 mg/kg | 30 mg/kg | 10 mg/kg | 30 mg/kg |
| Total leukocytes | 795000 | 488000 | 271000 | 39% | 66% |
| Macrophages | 443000 | 289000 | 172000 | 35% | 62% |
| Eosinophils | 335000 | 176000 | 91000 | 48% | 73% |

Airway Hyperresponsiveness

On day 24, three days after the challenge with OA aerosol, animals are anesthetized with pentobarbital sodium (100 mg/kg, i.p.) and tracheotomized (PE-190). A jugular vein is cannulated with a sylastic tubing for i.v. drug delivery. Animals are placed in a whole body plethysmograph with a built-in pneumotachograph and mechanically ventilated ($V_f$ =150/min., $V_t$=0.3ml; Model 683, Harvard Apparatus, S. Natic, MA) immediately following pancuronium bromide (0.1 mg/kg, i.v.) treatment. Tidal volume is obtained from an integration of the respiratory flow signal using a differential pressure transducer (Validyne DP 103-08, Northridge, CA). Transpulmonary pressure is measured with a differential pressure transducer (Validyne DP 45-30, Northridge. CA) as the difference between intratracheal pressure and intrapleural pressure (obtained from a cannula inserted into the intercostal space). Changes in lung resistance (cm $H_2O$ / ml / s) to increasing doses of methacholine (30, 100, 300, 1000 µg/kg, i.v.) are calculated from transpulmonary pressure, tidal volume, and respiratory flow measurements using a Modular Instrument Signal Processing System (Malvern, PA).

The results of the experiments indicate that retinoid antagonists can prevent or reverse allergic airway inflammation and inhibit antigen-induced bronchoconstriction, typical for allergic airway diseases, such as allergic bronchial asthma.

Example 7

Examples for the Effect of Retinoid Antagonists on Bone Resorption.

Bone Resorption Assay

A series of agents are known to induce or stimulate bone resorption. In this assay retinoid antagonists were tested for their capacity to inhibit or counteract the bone resorptive activity of bone resorption inducers.

Material and Methods.

Bone resorption was determined by quantification of calcium release from neonatal mouse calvaria (skull bones) in a tissue culture system, into supernatant medium. Calvaria were prepared from 4 days old mice (body weight 4-4.5 g) of time controlled mated Swiss albino mice. Frontal and parietal calvarial parts were dissected under a stereomicroscope and halved along the median suture. Half calvaria from all animals were randomly distributed to 6-well culture dishes (NUNC), containing stainless steel grids to support the bones at the interface of gas and medium. The tissue is incubated in BGJ medium (Bioconcept, Switzerland) according to a specified formulation supplemented with

1 mg/ml BSA (bovine serum albumin, SIGMA). Calvaria were cultured at 37°C in humidified atmosphere of 5% $CO_2$ and 95% air. After 24 hours of preincubation, they were transferred to new dishes containing 1.7 ml fresh medium and test substances. Bone resorption inducers and retinoid antagonists were tested either as single agents or in combination. The cultures were then run for 72 hours.
Calcium concentrations in culture supernatants were measured immediately after each incubation period. Stable, free calcium was determined in 25 μl samples with a spectrophotometric method using a methylthymol blue containing kit (Biomérieux), (E.M. Gindler and J.D. King, Am. J. Clin. Pathol. 1972, 58, 376-382). The resorptive response was expressed as μg calcium released per half calvarium during 3 days treatment.

Additional tests were done to examine calcium remaining in the calvaria. Calcium was extracted with 5% trichloroacetic acid (TCA) and determined in 50 μl samples after neutralization with NaOH. Furthermore, bone tissue viability was determined to exclude cytotoxicity. This was quantified by the colorimetric MTT tetrazolium test (T. Mosmann, J. Immunol. Methods 1983, 65, 55-63).

In these test, retinoid antagonists H and B were used.

The following bone resorption inducing/stimulating agents were used:

Calcitriol (1,25-dihydroxyvitamin $D_3$),
all-trans retinoic acid (all-trans RA), and
9-cis retinoic acid (9-cis RA), were synthesized by Roche Laboratories Basel.
Prostaglandin E2 (PGE2), cell culture tested (Sigma Chem. Co.),
Tumor necrosis factor $\alpha$ (TNF$\alpha$), murine recombinant, and
Interleukin-1$\alpha$ (IL-1$\alpha$), murine recombinant (Calbiochem.).

Bone resorption induced by the various agents was determined by measuring the amount of calcium released from half calvaria into the supernatant medium. Calcium release is indicated in μg per half calvarium in 1.7 ml medium. Calcium release from calvaria into medium as well as uptake of calcium from medium into calvaria can be measured. Efficacy of retinoid antagonists to inhibit the activity of bone resorption inducers is expressed as % inhibition. It is calculated from the difference between the amount of released calcium by bone resorption inducers alone and the amount of released calcium by their combination with retinoid antagonists. Retinoid antagonists alone induce neither calcium release nor uptake. Basal resorption rate in vehicle controls was 5 or less than 5%.

Results.

The results of the experiments (tables 6 to 11) demonstrate that retinoid antagonists H and B counteract bone resorption induced by six different agents. These latter are considered to contribute to bone resorption responsible for osteoporosis and bone fractures in mammals and human beings. As can be seen from tables 6 to 11, all six bone resorbing agents induced a dose dependent calcium release from calvaria bone into the supernatant medium. Retinoid antagonists were able to inhibit calcium release induced by these six different bone resorbing agents.

Inhibition of bone resorption by retinoid antagonists

Table 6: Calcitriol as inducing agent

| Bone resorption inducer Calcitriol (nM) | Retinoid antagonist H (µM) | Retinoid antagonist B (µM) | Calcium release per half calvarium (µg) H | Calcium release per half calvarium (µg) B | Inhibition of bone resorption by retinoid antagonists (%) H | Inhibition of bone resorption by retinoid antagonists (%) B |
|---|---|---|---|---|---|---|
| 3 | - | - | 13 ± 3 | | - | - |
| 1 | - | - | 9 ± 4 | | - | - |
| 0.3 | - | - | 4 ± 3 | | - | - |
| 3 | 1 | - | 11 ± 6 | | 15.4 | - |
| 1 | 1 | - | 4 ± 3 | | 55.6 | - |
| 0.3 | 1 | - | 0 ± 3 | | 100 | - |
| 3 | - | 1 | | 4 ± 4 | - | 69.2 |
| 1 | - | 1 | | 5 ± 3 | - | 44.4 |
| 0.3 | - | 1 | | -1 ± 2 | - | 125 |

Table 7: All-trans Retinoic Acid as inducing agent

| Bone resorption inducer all-trans RA (μM) | Retinoid antagonist H (μM) | Retinoid antagonist B (μM) | Calcium release per half calvarium (μg) H | Calcium release per half calvarium (μg) B | Inhibition of bone resorption by retinoid antagonist (%) H | Inhibition of bone resorption by retinoid antagonist (%) B |
|---|---|---|---|---|---|---|
| 1 | - | - | 23 ± 8 | 17 ± 3 | - | - |
| 0.1 | - | - | 19 ± 11 | 13 ± 3 | - | - |
| 0.01 | - | - | 1 ± 4 | 1 ± 2 | - | - |
| 1 | 10 | - | -2 ± 3 | | 108.7 | |
| 0.1 | 10 | - | -5 ± 4 | | 126.3 | |
| 0.1 | 1 | - | 2 ± 11 | | 89.5 | |
| 1 | - | 10 | | 10 ± 7 | | 41.2 |
| 0.1 | - | 10 | | -3 ± 4 | | 123.1 |
| 0.1 | - | 1 | | 5 ± 4 | | 61.5 |

Table 8: 9-cis Retinoic Acid as inducing agent

| Bone resorption inducer 9-cis RA (μM) | Retinoid antagonist H (μM) | Retinoid antagonist B (μM) | Calcium release per half calvarium (μg) H | Calcium release per half calvarium (μg) B | Inhibition of bone resorption by retinoid antagonists (%) H | Inhibition of bone resorption by retinoid antagonists (%) B |
|---|---|---|---|---|---|---|
| 1 | - | - | 40 ± 11 | 18 ± 4 | - | - |
| 0.1 | - | - | 22 ± 8 | 17 ± 10 | - | - |
| 0.01 | - | - | 6 ± 10 | 3 ± 3 | - | - |
| 1 | 10 | - | 28 ± 7 | | 30 | |
| 0.1 | 10 | - | -5 ± 3 | | 123 | |
| 0.1 | 1 | - | 4 ± 5 | | 82 | |
| 1 | - | 10 | | -6 ± 2 | | 133.3 |
| 0.1 | - | 10 | | -4 ± 3 | | 123.5 |
| 0.1 | - | 1 | | 2 ± 5 | | 33.3 |

Table 9: Prostaglandin E2 (PGE2) as inducing agent

| Bone resorption inducer PGE2 (µM) | Retinoid antagonist H (µM) | Retinoid antagonist B (µM) | Calcium release per half calvarium (µg) H | Calcium release per half calvarium (µg) B | Inhibition of bone resorption by retinoid antagonists (%) H | Inhibition of bone resorption by retinoid antagonists (%) B |
|---|---|---|---|---|---|---|
| 1 | - | - | 18 ± 5 | | - | - |
| 0.3 | - | - | 12 ± 4 | | - | - |
| 0.1 | - | - | 9 ± 5 | | - | - |
| 1 | 1 | - | 16 ± 4 | - | 11.1 | - |
| 0.3 | 1 | - | 8 ± 2 | - | 33.3 | - |
| 0.1 | 1 | - | 9 ± 8 | - | 0 | - |
| 1 | - | 1 | - | 15 ± 2 | - | 16.7 |
| 0.3 | - | 1 | - | 7 ± 4 | - | 41.7 |
| 0.1 | - | 1 | - | 2 ± 3 | - | 77.8 |

Table 10: Tumor Necrosis Factor α (TNF-α) as inducing agent

| Bone resorption inducer TNF-α (ng/ml) | Retinoid antagonist | | Calcium release per half calvarium (μg) | | Inhibition of bone resorption by retinoid antagonists (%) | |
|---|---|---|---|---|---|---|
| | H (μM) | B (μM) | H | B | H | B |
| 100 | - | - | 24 ± 3 | | - | - |
| 30 | - | - | 18 ± 8 | | - | - |
| 10 | - | - | 3 ± 4 | | - | - |
| 100 | 1 | - | 20 ± 4 | | 16.7 | |
| 30 | 1 | - | 20 ± 4 | | -11.1 | |
| 10 | 1 | - | 7 ± 6 | | -133.3 | |
| 100 | - | 1 | | 17 ± 3 | | 29.2 |
| 30 | - | 1 | | 12 ± 9 | | 33.3 |
| 10 | - | 1 | | 3 ± 4 | | 0 |

Table 11: Interleukin 1α (IL-1α) as inducing agent

| Bone resorption inducer IL-1α (nM) | Retinoid antagonist H (μM) | Retinoid antagonist B (μM) | Calcium release per half calvarium (μg) H | Calcium release per half calvarium (μg) B | Inhibition of bone resorption by retinoid antagonists (%) H | Inhibition of bone resorption by retinoid antagonists (%) B |
|---|---|---|---|---|---|---|
| 0.1 | - | - | 38 ± 5 | | - | - |
| 0.01 | - | - | 31 ± 9 | | - | - |
| 0.001 | - | - | 11 ± 10 | | - | - |
| 0.1 | 1 | - | 25 ± 6 | | 34.2 | |
| 0.01 | 1 | - | 24 ± 10 | | 19.4 | |
| 0.001 | 1 | - | 8 ± 9 | | 27.3 | |
| 0.1 | - | 1 | | 28 ± 8 | | 26.3 |
| 0.01 | - | 1 | | 26 ± 5 | | 16.1 |
| 0.001 | - | 1 | | 12 ± 6 | | -9.1 |

Example 8

Efficacy of topical application of Compound B in the treatment of precancerous lesions of the skin (multiple actinic keratoses)

In a clinical trial, a cream containing 1 % of Compound B was applied on the lesions twice daily without occlusive dressing. The results are given in Table 12. As can be seen from Table 12, Compound B when administered topically is effective in the therapy of precancerous lesions of the skin and is - in contrast to other retinoids- well tolerated without inducing any irritation of the lesions and the surrounding skin.

Table 12

| Patient | Sex | Age (Years) | Localisation | Duration of Therapy | Reduction in Size of Lesions | Improvement | Adverse Events * |
|---|---|---|---|---|---|---|---|
| I | m | 53 | both forearms | 4 months | 50 % | marked | none |
| II | m | 61 | face | 6 weeks | 20 % | slight | none |
| III | f | 69 | face | 4 months | 30 % | moderate | none |
| IV | m | 75 | face and back of both hands | 3 months | 30 % | moderate | none |
| V | m | 74 | face | 2 months | 0 % | no | none |

* particularly irritation of lesions and surrounding skin

In accordance with the present invention the compounds of formula I can be prepared by reacting a compound of formula XVIII

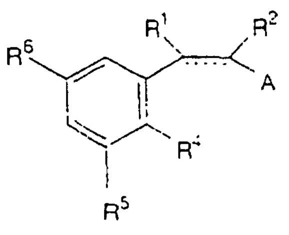

XVIII

with a compound of formula XIX

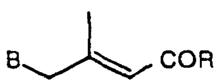

XIX

wherein A is formyl and B is di-(lower alkoxy)phosphinyl; R is lower alkoxy; and $R^1$, $R^2$ and $R^4$ to $R^6$ are as defined above;

to yield a compound of formula I wherein $R^3$ is lower alkoxy, and, if desired, hydrolysing the lower alkoxy group $R^3$ in the so obtained compound of formula I.

The reaction of the compound XVIII with the compound XIX can be carried out according to methods which are known per se for the Horner (Wadsworth-Emmons) reaction. The reaction can be carried out in the presence of a base and, preferably, in the presence of an inert organic solvent, e.g. in the presence of sodium hydride in benzene, toluene, dimethylformamide, tetrahydrofuran, dioxane or a 1,2-dimethoxyalkane, or also a sodium alcoholate in an alkanol, e.g. sodium methylate in methanol, in a temperature range lying between 0° and the boiling point of the reaction

mixture. Another example for a base which can be used in that reaction is lithium bis(trimethylsilyl)amide in an inert solvent like THF in a temperature range between -78°C and 0°C. A thus-obtained carboxylic acid ester of formula I can be hydrolyzed in a manner known per se, e.g. by treatment with alkalis, especially by treatment with aqueous-alcoholic sodium or potassium hydroxide solution in a temperature range lying between room temperature and the boiling point of the reaction mixture.

The thus-obtained carboxylic acid of formula I can be isolated in a manner known per se as such or as a salt, e.g. as an alkali salt, especially as the Na or K salt.

The compounds of formula XVIII are novel compounds and are also an object of the present invention. The compounds of formula XVIII can be prepared as set forth in Schemes 1 and 2 below:

Scheme 1

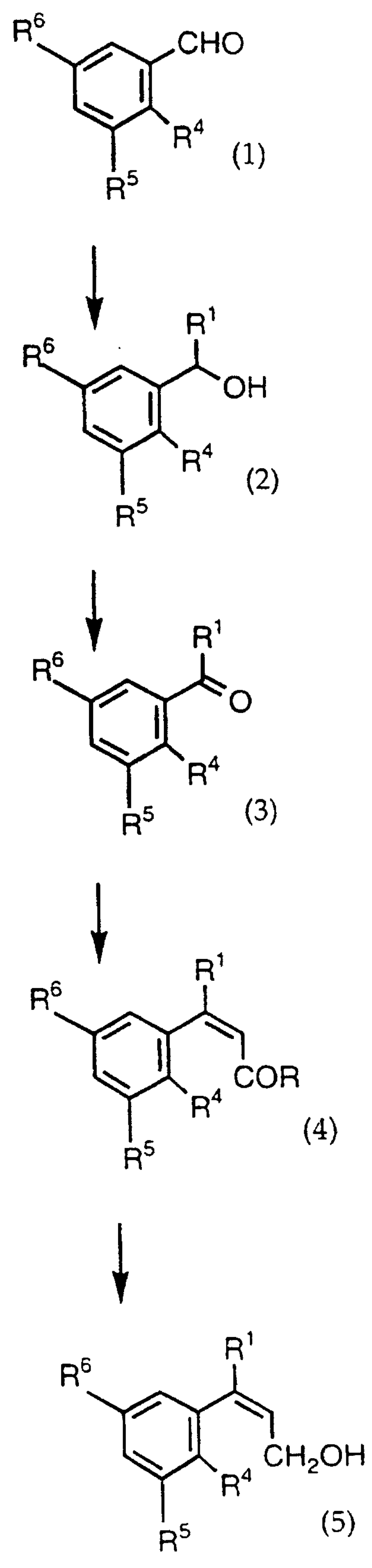

(1)

(2)

(3)

(4)

(5)

Scheme 2

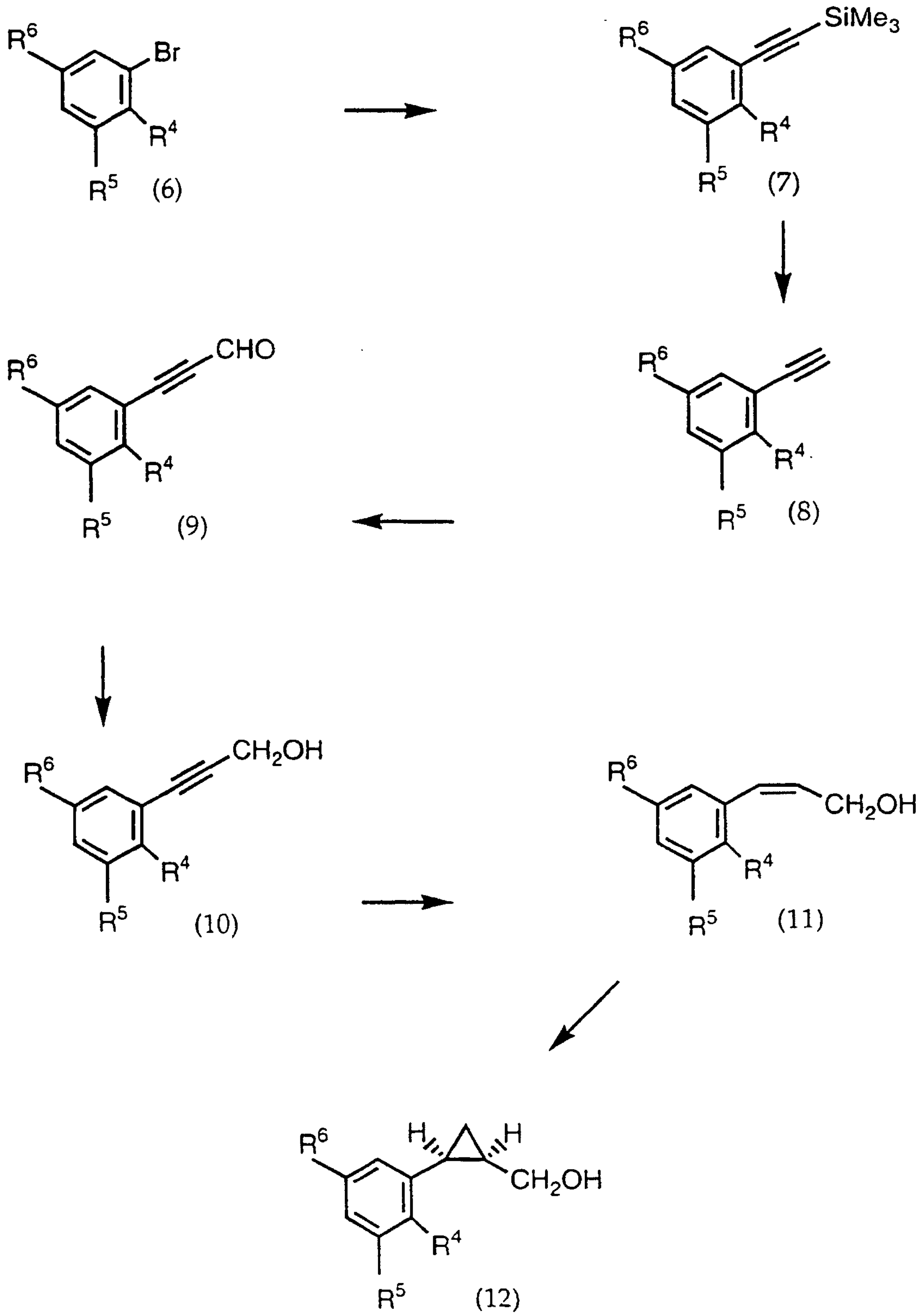

(6) (7) (8) (9) (10) (11) (12)

Compounds of formula XVIII wherein the dotted bond is present, $R^1$ is lower alkyl- and $R^2$ is hydrogen can be obtained according to Scheme 1. In Scheme 1, compound (1) is converted to compound (2) by reaction with an organometallic nucleophile like an alkyl lithium or alkyl magnesium halogenide. Compound (2) can be oxidized to form compound (3) by treatment with oxidizing agents such as manganese dioxide. Compound (3) can be converted to

compound (4) in a Peterson olefination [Synthesis, 384 (1984) D.J. Ager, J. Org. Chem. 33, 780 (1968) D.J. Peterson] by reaction with ethyl trimethylsilyl acetate. The carboxylic ester group in compound (4) can be reduced to a hydroxy methyl group by treatment with a metal hydride such as diisobutyl aluminum hydride to give compound (5) which can be oxidized by treatment with oxidizing agents such as manganese dioxide to give a compound of formula XVIII wherein $R^1$ is lower alkyl and $R^2$ is hydrogen.

Compounds of formula XVIII wherein the dotted bond is absent and $R^1$ and $R^2$ taken together are methylene can be obtained as set forth in Scheme 2. According to Scheme 2, compound (6) is reacted with trimethylsilyl acetylene in the presence of tetrakis (triphenylphosphine) palladium(0) and CuI to yield compound (7) which, after removal of the trimethylsilyl group by treatment with tetrabutyl ammonium fluoride and formylation of the so-obtained compound (8) with dimethyl formamide in the presence of a base such as butyl lithium yields compound (9). The formyl group in compound (9) can be reduced to the hydroxymethyl group by treatment with a metal hydride such as sodium borohydride in ethanol to give compound (10). Reduction of the triple bond in compound (10) e.g., by means of a Lindlar catalyst, affords compound (11) which is converted to compound (12) by a modified Simmons-Smith reaction [Tetrahedron 24, 53 (1968) J. Furukawa, N. Kawabata, J. Nishimura, J. Org. Chem. 42, 3031 (1977) N. Kawabata et al.]. This cyclopropanation can also be carried out in an enantioselective way according to the method of Fujisawa et al. (Chem. Letters, 61 (1992) using (R,R) or (S,S)-diethyltartrate as chiral auxiliary. Compound (12) can be oxidized using methods known in the art, e.g., by pyridinium chlorochromate, or by a Swern- or Dess-Martin oxidation to yield a compound of formula XVIII wherein A is formyl, the dotted bond is absent and $R^1$ and $R^2$ taken together are methylene.

All these reactions can be carried out in a manner known per se.

The invention is illustrated further by the Examples which follow.

Example 9

4.0 g of 3,5-di-tert.-butyl-2-hydroxybenzaldehyde (CAS # 37942-07-7) was dissolved in dimethylformamide (DMF). This was treated with 0.89 g of sodium hydride (50% in oil suspension) and stirred at 25°C until hydrogen was no longer evolved. To this was added 2.01 ml n-bromobutane and the mixture was heated to 85°C for 14 hr. This was cooled and poured into water. The aqueous was extracted with ether. The ether layer was washed with water, dried ($MgSO_4$), and solvent was removed. Purification was accomplished by florsil column chromatography (15% ether/hexane) to give 5.4 g of 3,5-di-tert.-butyl -2-butyloxybenzaldehyde; $^1H$ NMR ($CDCl_3$) $\delta$ 10.31 (s,1H,aldehyde), 7.70 (dd,4H,aromatics), 3.94 (t,2H,$-OCH_2-$).

3.5 g of 3,5- di-tert.-butyl-2-butyloxybenzaldehyde was dissolved in 30 ml of ethyl ether and cooled with stirring to 0°C. 11.4 ml of methyl lithium (1.55M in ether) was added via syringe. This was stirred for 10 min. and poured into 1M ammonium chloride and shaken. The ether layer was separated, dried ($Na_2SO_4$), and solvent removed to give 3.7 g of an oil which was purified by chromatography (silica gel - 5% ether/hexane) to give 3.3 g of 1-(3,5- di-tert.-butyl-2-butyloxyphenyl)ethanol; $^1H$ NMR ($CDCl_3$) $\delta$ 7.34 (dd,4H,aromatics), 5.25 (m,1H,$-CH_3C\underline{H}-$), 3.85 (t,2H,$-OCH_2-$).

2.77 g (9 mmol) of 1-(3,5- di-tert.-butyl-2-butyloxyphenyl)ethanol was dissolved in 120 ml of toluene that had been treated with 14 g of manganese(IV)oxide. This was well stirred at 75°C for 5 hr. This was cooled and filtered through celite. Solvent was removed to give 2.27 g of 1-(3,5- di-tert.-butyl -2-butyloxyphenyl)ethanone. $^1H$ NMR ($CDCl_3$) $\delta$ 7.38 (dd,4H,aromatics), 2.62 (s,3H, $C\underline{H}_3C-$), 3.72 (t, 2H, $-OCH_2-$).

1.43 g of diisopropylamine in 20 ml of tetrahydrofuran (THF) was treated with 8.4 ml of n-butyl lithium (1.6M in hexane) at -20°C. This was cooled to -78°C and treated with 2.26 g ethyl trimethylsilylacetate. This was allowed to warm to 0°C and poured into water and extracted into hexane. The extract was washed with water, dried ($MgSO_4$), and had solvent removed to give an oil. Purification and separation of isomers was accomplished by silica gel chromatography (15% ether/hexane) to give 3.5 of (Z)-3-(3,5-di-tert.-butyl -2-butyloxyphenyl)-butenoic acid ethyl ester. $^1H$ NMR ($CDCl_3$) $\delta$ 7.05 (dd, 4H, aromatics), 5.90 (s, 1H, 2-H), 3.96 (q, 2H, $-OCH_2CH_3$), 3.70 (t, 2H, $-OCH_2-$), 2.21 (s, 3H).

0.9 g of (E)-3-(3,5- di-tert.-butyl -2-butyloxyphenyl)-butenoic acid ethyl ester was dissolved in 20 ml of dry ether. This was cooled to -78°C and treated with 6.0 ml of diisobutylaluminum hydride (1.0M in hexane). The temperature was allowed to warm to 0°C and was then treated with 20% aqueous Rochelle salt. This was stirred at 25°C for 1 hr. The organic fraction was separated, washed with water, dried ($Na_2SO_4$), and had solvent removed to give 0.78 g of (Z)-3-(3,5- di-tert.-butyl -2-butyloxyphenyl)-buten-1-ol.

$^1H$ NMR ($CDCl_3$) $\delta$ 7.08 (dd, 4H, aromatics), 5.90 (t, 1H, $C\underline{H}-OH$), 3.80 (t, 2H, $-OCH_2-$), 2.49 (t, 1H, $-O\underline{H}$), 2.21 (s, 3H).

0.66 g of (Z)-3-(3,5- di-tert.-butyl-2-butyloxyphenyl)-buten-1-ol was dissolved in 100 ml ether and cooled to 15°C. 6.6 g of manganese(IV)oxide slurried in 50 ml ether was added to the above solution. This was stirred at ambient for 3 hr. The mixture was filtered through celite and had the solvent removed. This gave 0.64 g of (Z)-3-(3,5- di-tert.-butyl-2-butyloxyphenyl)-buten-1-al. $^1H$ NMR ($CDCl_3$) $\delta$ 9.45 (d, 1H, aldehyde), 7.08 (dd, 4H, aromatics), 5.90 (t, 1H, $C\underline{H}-OH$), 3.80 (t, 2H, $-OCH_2-$), 2.49 (t, 1H, $-O\underline{H}$), 2.21 (s, 3H).

635 mg of triethyl-3-methyl-4-phosphonocrotonate (CAS # 41891-54-7) was dissolved in 5 ml THF, cooled to -78°C and treated with 2.2 ml (2.2 mmol) (1.0M in THF) of lithium bis(trimethylsilyl)amide. While at -78°C, 600 mg of

(Z)-3-(3,5- di-tert.-butyl-2-butyloxyphenyl)-buten-1-al in 5 ml THF was slowly added. This was stirred at -78°C for 0.5 hr. and poured into dilute aqueous ammonium chloride. The product was extracted into hexane and the organic portion washed with water, dried ($Na_2SO_4$), and had the solvent removed to give a crude oil. Purification and isomer separation was accomplished by silica gel chromatography (3% ether/hexane) to give 540 mg of (2E,4E,6Z)-7-(3,5-di-tert.-butyl-2-butyloxyphenyl)-3,7-dimethyl-2,4,6-heptatrienoic acid ethyl ester.
$^1$H NMR ($CDCl_3$) δ 7.12 (dd, 4H, aromatics), 6.62 (dd, 1H, H-5), 6.20 (d, 1H,H-4), 6.18 (d, 1H,H-6), 4.15 (q, 3H, -$OCH_2$ C$\underline{H}_3$), 3.7 (dt, 2H, -$OCH_2$-), 2.22 (s, 3H), 2.15 (s, 3H).

520 mg of (2E,4E,6Z)-7-(3,5-di-tert.-butyl -2-butyloxyphenyl)-3,7-dimethyl-2,4,6-heptatrienoic acid ethyl ester was suspended in 5 ml ethanol and treated with 5 ml 6N NaOH and refluxed for 1.5 hr. This was cooled and acidified to pH 3 with dilute HCl. The solid which precipitated was extracted into chloroform. The organic portion was washed with water, dried ($Na_2SO_4$), and had the solvent removed. This gave a solid which was crystallized from methylene chloride/hexane to give (2E,4E,6Z)-7-[3,5-di-tert.butyl-2-butyloxyphenyl)-3-methyl-2,4,6-octatrienoic acid. $^1$H NMR ($CDCl_3$) δ 7.08 (dd, 4H, aromatics), 6.65 (dd, 1H,H-5), 6.22 (d, 1H,H-4), 6.18 (d, 1H,H-6), 3.7 (dt, 2H, -$OCH_2$-), 2.24 (s, 3H), 2.15 (s, 3H). M.p. 148-151°C.

In analogy to the above procedure the following compounds were prepared:

(2E,4E,6Z)-7-[3,5-di-tert.butyl-2-methoxyphenyl]-3-methyl-2,4,6-octatrienoic acid, m.p. 208-211°C (from THF/hexane)
(2E,4E,6Z)-7-[3,5-di-tert.butyl-2-ethoxyphenyl]-3-methyl-2,4,6-octatrienoic acid, m.p. 165-167°C (from THF/hexane)
(2E,4E,6Z)-7-[3,5-di-tert.butyl-2-hexyloxyphenyl]-3-methyl-2,4,6-octatrienoic acid, m.p. 156-160°C (from ether/hexane)
(2E,4E,6Z)-7-[3,5-di-tert.butyl-2-octyloxyphenyl)-3-methyl-2,4,6-octatrienoic acid, m.p. 137-139°C (from hexane).

Example 10

A solution of 13 g of 2-bromo-4,6-di-tert.butyl-phenol in 100 ml of dimethylformamide (DMF) was added dropwise to a suspension of 2.2 g of sodium hydride (50% in mineral oil) in 100 ml of DMF at 0°C. The reaction mixture was stirred at room temperature until hydrogen was no longer evolved (ca. 1 h), cooled again to 0°C and treated with a solution of 22 g of ethyl iodide in 50 ml of DMF. After stirring for about 1 hour at room temperature, the reaction mixture was poured on icewater, extracted with ethyl acetate, washed ($H_2O$), dried ($Na_2SO_4$) and evaporated to give 13.5 g of 1-bromo-2-ethoxy-3,5-di-tert.butyl-benzene as white crystals, m.p. 55-56°C.

10.1 g of 1-bromo-2-ethoxy-3,5-di-tert.butyl-benzene were dissolved in 50 ml of piperidine. After the addition of 490 mg of tetrakis(triphenylphosphine)palladium(0), 96 mg of copper(I)iodide and 140 mg of triphenylphosphine, the reaction mixture was heated to 90°C under argon and slowly treated with 6.3 g of (trimethylsilyl)acetylene (ca. 2 hours). The reaction was kept for further 10 minutes at 90°C before this procedure was repeated using the same amount of reagents. The reaction mixture was heated for another hour to 90°C, then poured on icewater, acidified with 80 ml of concentrated hydrochloric acid, extracted with ethyl acetate, washed ($H_2O$), dried ($Na_2SO_4$) and evaporated. The resulting oil was purified by chromatography (silica gel, hexane/2.5% ethyl acetate) to give 8.3 g of 3,5-di-tert. butyl-2-ethoxy-phenylethynyl)-trimethylsilane as a slightly yellow oil.

8.3 g of this oil were dissolved in 120 ml of tetrahydrofuran (THF) and treated with 6.03 g of tetrabutylammonium fluoride. After 1 hour of stirring at ambient temperature, the reaction mixture was poured on icewater, extracted with ether, washed ($H_2O$), dried ($Na_2SO_4$) and evaporated. Purification of the resulting brown oil by chromatography (silica gel, hexane) gave 4.2 g of 1,5-di-tert.butyl-2-ethoxy-3-ethynyl-benzene as colourless oil which crystallized in the refrigerator, m.p. 46-47°C. A small sample was sublimed in high vacuum and melted at 48-49°C.

4.1 g of this compound were dissolved in 45 ml of THF and treated with 11 ml of n-BuLi (1.6 molar in hexane) at -78°C. After 1 hour at -78°C the reaction mixture was treated with 12 ml of DMF, warmed to room temperature, stirred for 6 hours, poured on icewater, acidified with concentrated hydrochloric acid, extracted with ethyl acetate, washed ($H_2O$), dried ($Na_2SO_4$) and evaporated to give 5 g of a brown oil which was purified by chromatography (silica gel, hexane/2% ether). The combined pure fractions yielded 2.4 g of a slightly orange oil, which crystallized in the freezer, m.p. 55-57°C.

2.4 g of 3,5-di-tert.butyl-2-ethoxy-phenyl)-propynal were dissolved in 25 ml of ethanol and treated with 90 mg of sodium borohydride during 1.5 hours at 0°C. The reaction mixture was acidified with 6 ml of 2N hydrochloric acid, poured on icewater, extracted with ether, washed ($H_2O$), dried ($Na_2SO_4$) and evaporated. The resulting orange oil was purified by chromatography (silica gel, hexane:ethyl acetate = 9:1) and recrystallized from pentane to give 2.2 g of 3-(3,5-di-tert.butyl-2-ethoxy-phenyl)-prop-2-yn-1-ol as white crystals, m.p. 82-84°C.

2 g of this compound were dissolved in 350 ml of ethanol, treated with 100 mg of Lindlar-catalyst and hydrogenated at normal pressure for 7.5 hours. After 3, 5 and 6 hours, 100 mg each of fresh Lindlar-catalyst were added.

The catalyst was filtered off and the reaction solution evaporated. The resulting residue was purified by medium pressure chromatography (silica gel, hexane:ether = 8:2) and gave after crystallisation from hexane 1.3 g of (Z)-3-(3,5-di-tert.butyl-2-ethoxy-phenyl)-prop-2-en-1-ol, mp. 93-94°C.

1.3 g of this compound were dissolved in 50 ml of methylene chloride and treated with 13.7 ml of diethylzinc (1M in hexane) at -5°C. After 15 minutes of stirring at 0°C, the reaction mixture was cooled to -20°C, treated dropwise with 7.4 g of methylene iodide, stirred for 1 hour at 0°C and 1.5 hours at room temperature and recooled to 0°C. 50 ml of saturated, aqueous ammonium chloride were dropped slowly into the white suspension. The clear reaction mixture was poured onto ice/saturated ammonium chloride solution, extracted with ether, washed ($H_2O$), dried ($Na_2SO_4$) and evaporated. The semi-crystalline residue was chromatographed (silica gel, hexane:ethyl acetate = 9:1) and gave after recrystallisation from hexane 1.3 g of (1RS,2SR)-[2-(3,5-di-tert.butyl-2-ethoxy-phenyl)-cyclopropyl]-methanol as white crystals, m.p. 102-103°C.

1.02 g of oxalyl chloride were dissolved in 37 ml of $CH_2Cl_2$ and treated with 0.86 ml of dimethyl sulfoxide at -60°C. After shortly warming to -35°C, the reaction mixture was recooled to -60°C and treated with a solution of 1.2 g of (1RS,2SR)-[2-(3,5-di-tert.butyl-2-ethoxy-phenyl)-cyclopropyl]-methanol in 20 ml of $CH_2Cl_2$. After 15 minutes of stirring at -50°C, 1.7 ml of triethylamine were dropped in. The reaction mixture was stirred for 2 hours at room temperature, poured on icewater, extracted with ether, washed ($H_2O$), dried ($Na_2SO_4$) and evaporated. The crude material was purified by flash chromatography (silica gel, hexane:ethyl acetate = 9:1) and recrystallized from hexane to give 1.01 g of (1RS,2RS)-2-(3,5-di-tert.butyl-2-ethoxy-phenyl)-cyclopropanecarbaldehyde as white crystals, m.p. 96-97°C.

908 mg of triethyl 3-methyl-4-phosphonocrotonate were dissolved in 25 ml of THF and treated with 3.4 ml of lithium bis(trimethylsilyl)amide (1.0 molar in THF) at -78°C. After 0.5 hour, a solution of 800 mg of the above described aldehyde in 25 ml of THF was slowly added. The cooling bath was removed and the temperature allowed to warm to 0°C. After 1.5 hours at 0°C, the reaction mixture was poured on saturated, aqueous ammonium chloride, extracted with ether, washed ($H_2O$), dried ($Na_2SO_4$) and evaporated. The crude product was purified by flash chromatography (silica gel, hexane:ethyl acetate = 9:1) and medium pressure chromatography (silica gel, hexane/2% ethyl acetate) to give after recrystallisation from hexane/ethyl acetate 650 mg of (2E,4E)-(1RS,2RS)-5-[2-(3,5-di-tert.butyl-2-ethoxy-phenyl)-cyclopropyl]-3-methyl-penta-2,4-dienoic acid ethyl ester as white crystals, m.p. 129-130°C and 370 mg of (2Z,4E)-(1RS,2RS)-5-[2-(3,5-di-tert.butyl-2-ethoxy-phenyl)-cyclopropyl]-3-methyl-penta-2,4-dienoic acid ethyl ester, m.p. 123-126°C.

600 mg of the (2E,4E)-ester were dissolved in 20 ml of ethanol and treated with a solution of 840 mg of potassium hydroxide in 4 ml each of ethanol and water. After the addition of 10 ml of THF, the clear solution was warmed to 45-50°C for 7 hours, cooled to room temperature, poured in ice/1N hydrochloric acid, extracted with ethyl acetate, washed ($H_2O$), dried ($Na_2SO_4$) and evaporated. The crude material was recrystallized from ethyl acetate to give 440 mg of (2E,4E)-(1RS,2RS)-5-[2-(3,5-di-tert.butyl-2-ethoxyphenyl)-cyclopropyl]-3-methyl-penta-2,4-dienoic acid as white crystals, m.p. 200-202°C. According to the same procedure 370 mg of the (2Z,4E)-ester were hydrolized to give 270 mg of (2Z,4E)-(1RS,2RS)-5-[2-(3,5-di-tert.butyl-2-ethoxy-phenyl)-cyclopropyl]-3-methyl-penta-2,4-dienoic acid, m.p. 169-174°C.

Example 11

In analogy to Example 2, (2E,4E)-(1RS,2RS)-5-[2-(3,5-di-tert.butyl-2-butoxy-phenyl)-cyclopropyl]-3-methyl-penta-2,4-dienoic acid, m.p. 175-178°C (hexane/ethyl acetate) was synthesized using 1-bromo-2-butoxy-3,5-di-tert. butyl-benzene as starting material.

The following Examples describe pharmaceutical formulations according to the invention:

Example 12

a) Fill mass for soft gelatin capsules

| | |
|---|---|
| Active compound | 5.0-200.0 g |
| Oil* | 1-3 parts |
| Wax mixture ** | 1-5 parts |
| Fill volume | 1-6 minims |

* natural vegetable oils, e.g. soy oil, peanut oil, and artificial glycerides

** composition of natural and artificial waxes or partially hydrogenated fats

b) 20 mg Soft Gelatin Capsules

| Ingredients | mg/capsule |
|---|---|
| Active compound | 20.000 |
| dl-α-Tocopherol | 0.028 |
| Hydrogenated Castor Oil | 4.200 |
| Caprylic/Capric/Stearic Triglyceride (Synthetic Triglyceride) | 56.000 |
| Triglyceride, Medium Chain | 199.772 |
| | Total 280.000 mg |

Example 13

Hard Gelatine capsules containing 20 mg active substance:

| Composition: One Capsule contains: | |
|---|---|
| Active compound | 20.0 mg |
| Gelatine Bloom 30 | 70.0 mg |
| Maltodextrin MD 05 | 108.0 mg |
| dl-α-Tocopherol | 2.0 mg |
| Sodium ascorbate | 10.0 mg |
| Microcrystalline cellulose | 48.0 mg |
| Magnesium stearate | 2.0 mg |
| (weight capsule content) | 260.0 mg |

Procedure:

The active substance is wet milled in a solution of gelatine, maltodextrin, dl-□-Tocopherol and sodium ascorbate.
The wet milled suspension is spray-dried.
The spray-dried powder is mixed with microcrystalline cellulose and magnesium stearate.
260 mg each of this mixture are filled into hard gelatine capsules of suitable size and color.

Example 14

Tablet containing 20 mg active substance:

| Tablet kernel: | |
|---|---|
| Active compound | 20.0 mg |
| Anhydrous lactose | 130.5 mg |
| Microcrystalline Cellulose | 80.0 mg |
| dl-α-Tocopherol | 2.0 mg |
| Sodium ascorbate | 10.0 mg |
| Polyvinylpyrrolidone K30 | 5.0 mg |
| Magnesium stearate | 2.5 mg |
| (Kernel weight) | 250.0 mg |
| Film coat: | |
| Hydroxypropyl methylcellulose | 3.5 mg |
| Polyethytenglycol 6000 | 0.8 mg |
| Talc | 1.3 mg |
| Irone oxide, yellow | 0.8 mg |
| Titanium dioxide | 0.8 mg |
| (weight of the film) | 7.4 mg |

Procedure:

The compound is mixed with anhydrous lactose and microcrystalline cellulose.
The mixture is granulated in water with a solution/dispersion of polyvinylpyrrolidone, dl-α-Tocopherol and sodium ascorbate.
The granular material is mixed with magnesium stearate and afterwards pressed as kernels with 250 mg weight.
The kernels are film coated with a solution/suspension of above-mentioned compositions.

Example 15

| Sachet containing active substance | |
|---|---|
| Active compound | 200.0 mg |
| Lactose, fine powder | 990.0 mg |
| Microcrystalline Cellulose | 1250.0 mg |
| Sodium Carboxymethyl cellulose | 14.0 mg |
| dl-α-Tocopherol | 5.0 mg |
| Sodium ascorbate | 20.0 mg |
| Polyvinylpyrrolidone K30 | 10.0 mg |
| Magnesium stearate | 10.0 mg |

Example 16

| Lotion (solution) | | preferred |
|---|---|---|
| Active compound | 0.1-2.0 g | |
| Propylene Glycol | 5.00-20.00 g | 10.00 g |
| PEG-Glyceryl Cocoate * | 0.00-20.00 g | 10.00 g |
| dl-α-Tocopherol | 0.001-0.50 g | 0.02 g |
| Ascorbyl Palmitate | 0.01-0.20 g | 0.10 g |
| Propyl Gallate | 0.001-0.02 g | 0.002 g |

*or other tensides

(continued)

| Lotion (solution) | | preferred |
|---|---|---|
| Citric acid, anhydr.** | 0.00-0.20 g | 0.01 g |
| Isopropanol *** | 40.00-90.00 g | 50.00 g |
| Water, dem. ad | 100.00 g | 100.00 g resp. ml |

**or other complexing agents e.g. EDTA

***or other alcohols e.g. Ethanol

Examnle 17

| Gel | | |
|---|---|---|
| | | preferred |
| Active compound | 0.1-2.0 g | |
| Propylene Glycol | 5.00-20.00 g | 10.00 g |
| PEG-Glyceryl Cocoate * | 0.00-20.00 g | 10.00 g |
| dl-α-Tocopherol | 0.001-0.50 g | 0.02 g |
| Ascorbyl Palmitate | 0.01-0.20 g | 0.10 g |
| Propyl Gallate | 0.001-0.02 g | 0.002 g |
| Citric acid, anhydr. ** | 0.00-0.20 g | 0.01 g |
| Isopropanol *** | 40.00-90.00 g | 50.00 g |
| HPMC **** | 0.50-5.00 g | 3.00 g |
| Preservative ***** | q.s. | q.s. |
| Water, dem. ad | 100.00 g | 100.00 g resp. ml |

* or other tensides

** or other complexing agents e.g. EDTA

*** or other alcohols e.g. Ethanol

**** Hydroxypropyl Methylcellulose or other polymers e.g. neutralised Carbomer, Methyl Cellulose, Sodium Carboxymethylcellulose

***** Preservatives e.g., Paraben esters (methyl, ethyl, propyl, butyl). Sorbic Acid. Benzoic Acid

Example 18

| Cream | | |
|---|---|---|
| | preferred | |
| Active compound | 0.1-2.0 g | |
| Glycerol | 0.00-10.00 g | 5.00 g |
| $Na_2$ EDTA | 0.001-0.50 g | 0.03 g |
| Glycerides * | 5.00-20.00 g | 10.00 g |
| Cetyl Alcohol | 0.50-5.00 g | 1.00 g |
| Stearyl Alcohol | 0.50-5.00 g | 1.00 g |
| Glycerol mono Stearate | 1.00-8.00 g | 4.00 g |
| Ceteareth ** | 0.50-5.00 g | 2.00 g |
| dl-α-Tocopherol | 0.001-0.50 g | 0.02 g |
| Preservative *** | q.s. | q.s. |

* e.g. Caprylic/Capric/Triglyceride, Caprylic/Capric/Linoleic Triglycerides, natural glycerides, as well as e.g. Propylene Glycol, Dicaprylate/Dicaprate and waxes, such as Stearyl, Stearate, Oleyl Oleate, Isopropyl Myristate

** Ceteareth 5-30, or other emulsifiers such as Polysorbase 20-80, Sorbitane esters of fatty acids, fatty acid esters of PEG.

*** Preservatives e.g., Paraben esters (methyl, ethyl, propyl, butyl). Sorbic Acid. Benzoic Acid

(continued)

| Cream | | |
|---|---|---|
| | preferred | |
| Water, dem. ad | 100.00 g | 100.00 g |

Example 19

| Aerosol for inhalation, metered dose inhaler | |
|---|---|
| Active compound | 0.5 % (0.1-2.0%) |
| Sorbitantrioleate | 5 % |
| dl-α-Tocopherol | 0.4% |
| Propellant (mixture of Trichlorofluoromethane and Dichlorodifluoromethane) | 94.1 % |

.Example 20

| Dry powder inhaler | |
|---|---|
| Active compound (jet-milled, spray-dried) | 0.5 mg (0.1 mg - 2.0 mg) |
| Lactose monohydrate | 25 mg |

**Claims**

1. Compounds of the formula I

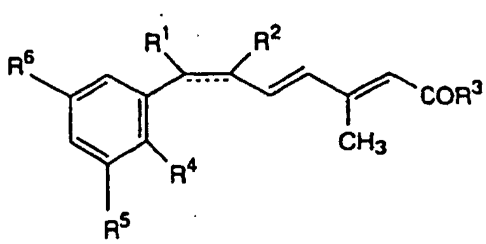

I

wherein the dotted bond is optional; and, when the dotted bond is present, $R^1$ is alkyl containing from 1 to 7 carbon atoms and $R^2$ is hydrogen; and, when the dotted bond is absent, $R^1$ and $R^2$ taken together are methylene to form a cis-substituted cyclopropyl ring; $R^3$ is hydroxy or lower alkoxy; $R^4$ is alkyl or alkoxy; and $R^5$ and $R^6$ are, independently, a $C_{4-12}$ alkyl group or a mono- or polycyclic $C_{5-12}$-hydrocarbon group which are linked to the phenyl ring through a quaternary carbon atom,
and pharmaceutically acceptable salts of carboxylic acids of formula I.

2. Compounds as in claim 1 of the formula

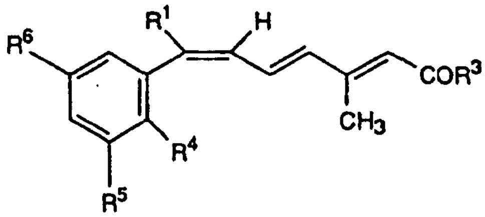

I a

wherein $R^1$ is alkyl containing from 1 to 7 carbon atoms and $R^3$ to $R^6$ are as in claim 1;
and pharmaceutically acceptable salts of carboxylic acids of formula Ia.

**3.** The compound of claim 2, (2E,4E,6Z)-7-[3,5-di-tert.butyl-2-ethoxyphenyl]-3-methyl-2,4,6-octatrienoic acid.

**4.** The compound of claim 2, (2E,4E,6Z)-7-[3,5-di-tert.butyl-2-butyloxyphenyl]-3-methyl-2,4,6-octatrienoic acid.

**5.** The compounds of claim 2,
(2E,4E,6Z)-7-[3,5-di-tert.butyl-2-butyloxyphenyl]-3-methyl-2,4,6-octatrienoic acid ethyl ester,
(2E,4E,6Z)-7-[3,5-di-tert.butyl-2-methoxyphenyl]-3-methyl-2,4,6-octatrienoic acid,
(2E,4E,6Z)-7-[3,5-di-tert.butyl-2-hexyloxyphenyl]-3-methyl-2,4,6-octatrienoic acid,
(2E,4E,6Z)-7-[3,5-di-tert.butyl-2-octyloxyphenyl]-3-methyl-2,4,6-octatrienoic acid.

**6.** Compounds as in claim **1**, of the formula

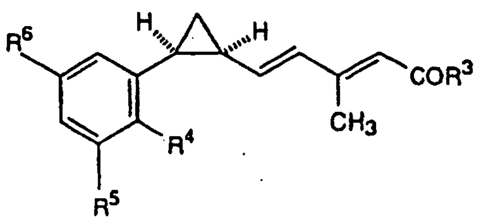

Ib

wherein $R^3$ to $R^6$ are as in claim 1;
and pharmaceutically acceptable salts of carboxylic acids of formula Ib.

**7.** The compound of claim 6,
(2E,4E)-(1RS,2RS)-5-[2-(3,5-di-tert.butyl-2-ethoxy-phenyl)-cyclopropyl]-3-methyl-penta-2,4-dienoic acid ethyl ester,
(2E,4E)-(1RS,2RS)-5-[2-(3,5-di-tert.butyl-2-ethoxy-phenyl)-cyclopropyl]-3-methyl-penta-2,4-dienoic acid.
(2E,4E)-(1RS,2RS)-5-[2-(3,5-di-tert.butyl-2-butoxy-phenyl)-cyclopropyl]-3-methyl-penta-2,4-dienoic acid.

**8.** Compounds of formula

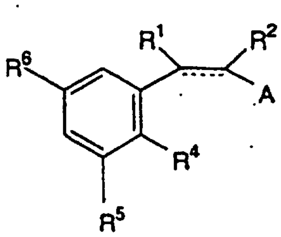

XVIII

wherein A is formyl; and $R^1$, $R^2$ and $R^4$ to $R^6$ are as defined in claim 1, and the dotted bond is optional.

**9.** Pharmaceutical preparations, containing a compound of formula I of claim 1 or pharmaceutically acceptable salts of a carboxylic acid of formula I and usual pharmaceutical carrier materials.

**10.** The use of compounds of formula I of claims 1-7, or pharmaceutically acceptable salts of a carboxylic acid of formula I in the manufacture of a medicament for reducing or abolishing adverse events in patients receiving retinoid agonist treatment.

**11.** The use of compounds of formula I of claims 1-7, or pharmaceutically acceptable salts of a carboxylic acid of formula I as in claim 10, wherein the retinoid agonist treatment is for acne, psoriasis or premalignant or malignant diseases.

**12.** The use of compounds of formula I of claims 1-7, or pharmaceutically acceptable salts of a carboxylic acid of formula I in the manufacture of a medicament for the treatment of T-helper cell type 2 (Th2)-mediated immune diseases, such as immunoglobulin E (IgE)-mediated allergic diseases or for the treatment of diseases mediated byTh2-related cytokines such as IL-4 and IL-5.

**13.** The use as in claim 12 wherein the disease is atopic dermatitis, allergic rhinitis or allergic bronchial asthma.

**14.** The use of compounds of formula I of claims 1-7, or pharmaceutically acceptable salts of a carboxylic acid of formula I in the manufacture of a medicament for the treatment of osteoporosis.

**15.** The use as in claim 14 wherein the osteoporosis is postmenopausal osteoporosis, or senile osteoporosis in both sexes, or all forms of secondary osteoporosis.

**16.** The use of compounds of formula I of claims 1-7 or pharmaceutically acceptable salts of a carboxylic acid of formula I in the manufacture of a medicament for the treatment of preneoplastic or neoplastic diseases.

**17.** The use as in claim 16 wherein the preneoplastic disease is precancerous lesions of the shin and mucous membranes, and the neoplastic disease is solid tumors of skin, mucous membranes, head and neck, lung, stomach, colon, breast, ovary, cervix and prostate.

**18.** The use as in any one of claims 10-17, wherein the retinoic acid antagonist is a compound of formula I as claimed in claim 1.

**19.** The use as in claim 18, wherein the compound of formula I is a compound of formula Ic

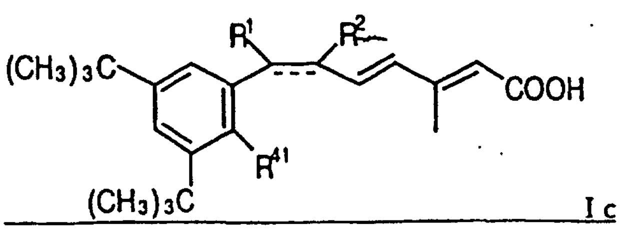

I c

wherein the dotted bond is optional; and, when the dotted bond is present, $R^1$ is methyl and $R^2$ is hydrogen; and, when the dotted bond is absent, $R^1$ and $R^2$ taken together are methylene to form a cis-substituted cyclopropyl ring; and $R^{41}$ is $C_{1-4}$-alkoxy.

**20.** The use as in claim 19, wherein the compound of formula I c is (2E,4E)-(1RS,2RS)-5-[2-(3,5-di-tert.butyl-2-butoxy-phenyl)-cyclopropyl]-3-methyl-penta-2,4-dienoic acid.

**Patentansprüche**

**1.** Verbindungen der Formel I

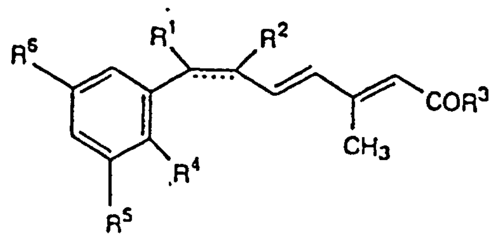

I

worin die gepunktete Bindung fakultativ ist; und, wenn die gepunktete Bindung vorliegt, $R^1$ Alkyl, das von 1 bis 7 Kohlenstoffatome enthält, ist und $R^2$ Wasserstoff ist; und, wenn die gepunktete Bindung fehlt, $R^1$ und $R^2$ zusammen genommen Methylen sind, so dass diese einen cis-substituierten Cyclopropylring bilden; $R^3$ Hydroxy oder niederes Alkoxy ist; $R^4$ Alkyl oder Alkoxy ist; und $R^5$ und $R^6$ unabhängig eine $C_{4-12}$-Alkylgruppe oder eine mono- oder polycyclische $C_{5-12}$-Kohlenwasserstoffgruppe sind, welche mit dem Phenylring über ein quarternäres Kohlenstoffatom verknüpft sind,
sowie pharmazeutisch verträgliche Salze von Carbonsäuren der Formel I.

**2.** Verbindungen wie in Anspruch 1 mit der Formel

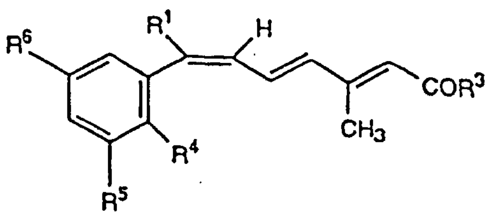

Ia

worin $R^1$ Alkyl, das von 1 bis 7 Kohlenstoffatome enthält, ist und $R^3$ bis $R^6$ wie in Anspruch 1 sind,
sowie pharmazeutisch verträgliche Salze von Carbonsäuren der Formel Ia.

**3.** Die Verbindung nach Anspruch 2 (2E,4E,6Z)-7-[3,5-Di-tert.-butyl-2-ethoxyphenyl]-3-methyl-2,4,6-octatriensäure.

**4.** Die Verbindung nach Anspruch 2 (2E,4E,6Z)-7-[3,5-Di-tert.-butyl-2-butyloxyphenyl]-3-methyl-2,4,6-octatriensäure.

**5.** Die Verbindungen nach Anspruch 2
(2E,4E,6Z)-7-[3,5-Di-tert.-butyl-2-butyloxyphenyl]-3-methyl-2,4,6-octatriensäureethylester,
(2E,4E,6Z)-7-[3,5-Di-tert.-butyl-2-methoxyphenyl]-3-methyl-2,4,6-octatriensäure, (2E,4E,6Z)-7-[3,5-Di-tert.-butyl-2-hexyloxyphenyl]-3-methyl-2,4,6-octatriensäure,
(2E,4E,6Z)-7-[3,5-Di-tert.-butyl-2-octyloxyphenyl]-3-methyl-2,4,6-octatriensäure.

**6.** Verbindungen wie in Anspruch 1 mit der Formel

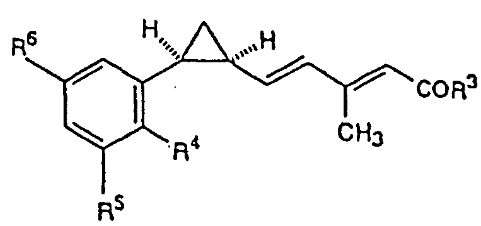

Ib

worin $R^3$ bis $R^6$ wie in Anspruch 1 sind;
sowie pharmazeutisch verträgliche Salze von Carbonsäuren der Formel Ib.

**7.** Die Verbindung nach Anspruch 6
(2E,4E)-(1RS,2RS)-5-[2-(3,5-Di-tert.-butyl-2-ethoxy-phenyl)-cyclopropyl]-3-methyl-penta-2,4-diensäure-ethylester,
(2E,4E)-(1RS,2RS)-5-[2-(3,5-Di-tert.-butyl-2-ethoxy-phenyl)-cyclopropyl]-3-methyl-penta-2,4-diensäure,
(2E,4E)-(IRS,2RS)-5-[2-(3,5-di-tert.-butyl-2-butoxy-phenyl)-cyclopropyl]-3-methyl-penta-2,4-diensäure.

**8.** Verbindungen der Formel

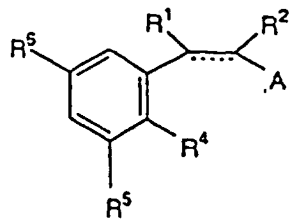

XVIII

worin A Formyl ist; und $R^1$, $R^2$ und $R^4$ bis $R^6$ wie in Anspruch 1 definiert sind, und die gepunktete Bindung fakultativ ist.

**9.** Pharmazeutische Präparate, enthaltend eine Verbindung der Formel I nach Anspruch 1 oder pharmazeutisch verträgliche Salze einer Carbonsäure der Formel I und übliche pharmazeutische Trägermaterialien.

**10.** Die Verwendung von Verbindungen der Formel I der Ansprüche 1 - 7 oder pharmazeutisch verträglicher Salze einer Carbonsäure der Formel I bei der Herstellung eines Medikaments zur Verringerung oder Beseitigung nachteiliger Auswirkungen bei Patienten, welche eine Behandlung mit Retinoid-Agonisten erhalten.

**11.** Die Verwendung von Verbindungen der Formel I der Ansprüche 1 - 7 oder pharmazeutisch verträglicher Salze einer Carbonsäure der Formel I wie in Anspruch 10, wobei die Behandlung mit Retinoid-Agonisten eine gegen Akne, Psoriasis oder prämaligne oder maligne Erkrankungen ist.

**12.** Die Verwendung von Verbindungen der Formel I der Ansprüche 1 - 7 oder pharmazeutisch verträglicher Salze einer Carbonsäure der Formel I bei der Herstellung eines Medikaments zur Behandlung von T-Helferzelltyp 2 (Th2)-vermittelten Immunkrankheiten wie beispielsweise Immunglobulin E (IgE)-vermittelten allergischen Erkrankungen oder zur Behandlung von Krankheiten, welche durch Th2-verwandte Cytokine wie z.B. IL-4 und IL-5 vermittelt werden.

**13.** Die Verwendung wie in Anspruch 12, wobei die Erkrankung atopische Dermatitis, allergische Rhinitis oder allergisches Bronchialasthma ist.

**14.** Die Verwendung von Verbindungen der Formel I der Ansprüche 1 - 7 oder pharmazeutisch verträglicher Salze einer Carbonsäure der Formel I bei der Herstellung eines Medikaments zur Behandlung von Osteoporose.

**15.** Die Verwendung wie in Anspruch 14, wobei die Osteoporose postmenopausale Osteoporose oder senile Osteoporose bei beiden Geschlechtern oder irgendeine Form von sekundärer Osteoporose ist.

**16.** Die Verwendung von Verbindungen der Formel I der Ansprüche 1 - 7 oder pharmazeutisch verträglicher Salze einer Carbonsäure der Formel I bei der Herstellung eines Medikaments zur Behandlung von präneoplastischen oder neoplastischen Erkrankungen.

**17.** Die Verwendung wie in Anspruch 16, wobei die präneoplastische Erkrankung präkanzeröse Schädigungen der Haut und der Schleimhäute sind, und die neoplastische Erkrankung solide Tumore der Haut, der Schleimhäute, des Kopfes und des Halses, der Lunge, des Magens, des Darms, der Brust, der Eierstöcke, des Zervix und der Prostata sind.

**18.** Die Verwendung wie in irgendeinem der Ansprüche 10 - 17, wobei der Retinsäure-Antagonist eine Verbindung der Formel I wie in Anspruch 1 beansprucht ist.

**19.** Die Verwendung wie in Anspruch 18, wobei die Verbindung der Formel I eine Verbindung der Formel Ic

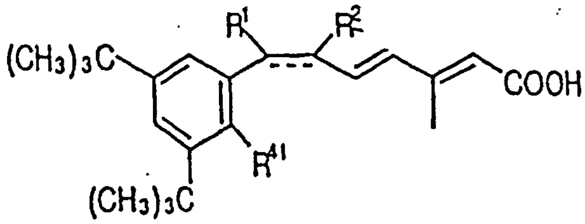

Ic

ist, worin die gepunktete Bindung fakultativ ist; und, wenn die gepunktete Bindung vorliegt, $R^1$ Methyl ist und $R^2$ Wasserstoff ist; und, wenn die gepunktete Bindung fehlt, $R^1$ und $R^2$ zusammen genommen Methylen sind, so dass diese einen cis-substituierten Cyclopropylring bilden; und $R^{41}$ $C_{1-4}$-Alkoxy ist.

**20.** Die Verwendung wie in Anspruch 19, wobei die Verbindung der Formel Ic (2E,4E)-(1RS,2RS)-5-[2-(3,5-Di-tert.-butyl-2-butoxy-phenyl)-cyclopropyl]-3-methyl-penta-2,4-diensäure ist.

**Revendications**

**1.** Composés de formule I :

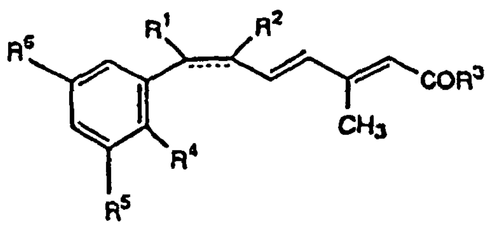

I

dans laquelle la liaison en pointillés est facultative; et, lorsque la liaison en pointillés est présente, $R^1$ est un alkyle contenant 1 à 7 atomes de carbone et $R^2$ est l'hydrogène; et, lorsque la liaison en pointillés est absente, $R^1$ et $R^2$, pris conjointement, sont du méthylène pour former un cycle cyclopropyle substitué en cis; $R^3$ est un hydroxy ou un alcoxy inférieur; $R^4$ est un alkyle ou un alcoxy; et $R^5$ et $R^6$ sont indépendamment un groupement alkyle en $C_{4-12}$ ou un groupement hydrocarboné mono- ou polycyclique en $C_{5-12}$ qui est lié au cycle phényle par un atome de carbone quaternaire, et sels pharmaceutiquement acceptables d'acides carboxyliques de formule I.

**2.** Composés selon la revendication 1 de formule :

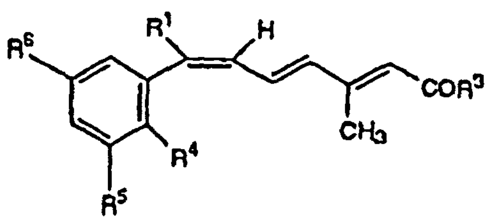

I a

dans laquelle $R^1$ est un alkyle contenant 1 à 7 atomes de carbone et $R^3$ à $R^6$ sont tels que définis dans la revendication 1, et sels pharmaceutiquement acceptables d'acides carboxyliques de formule Ia.

**3.** Composé selon la revendication 2, soit l'acide (2E,4E,6Z)-7-[3,5-di-tert.butyl-2-éthoxyphényl]-3-méthyl-2,4,6-octatriénoïque.

**4.** Composé selon la revendication 2, soit l'acide (2E,4E,6Z)-7-[3,5-di-tert.butyl-2-butyloxyphényl)-3-méthyl-2,9,6-octatriénoïque.

5. Composés selon la revendication 2, soit l'ester éthylique de l'acide (2E,4E,6Z)-7-[3,5-di-tert.butyl-2-butyloxyphényl]-3-méthyl-2,4,6-octatriénoïque, l'acide (2E,4E,6Z)-7-[3,5-di-tert.butyl-2-méthoxyphényl]-3-méthyl-2,4,6-octatriénoïque, l'acide (2E,4E,6Z)-7-[3,5-di-tert.butyl-2-hexyloxyphényl]-3-méthyl-2,4,6-octatriénoïque, et l'acide (2E,4E,6Z)-7-[3,5-di-tert.butyl-2-octyloxyphényl]-3-méthyl-2,4,6-octatriénoïque.

6. Composés selon la revendication 1 de formule :

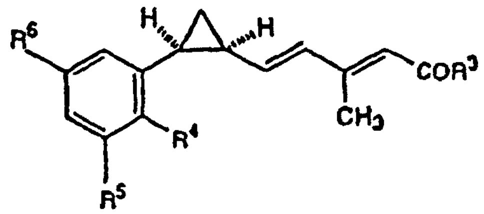

Ib

dans laquelle $R^3$ à $R^6$ sont comme dans la revendication 1, et sels pharmaceutiquement acceptable d'acides carboxyliques de formule Ib.

7. Composé selon la revendication 6, soit l'ester éthylique de l'acide(2E,4E)-(1RS,2RS)-5-[2-(3,5-di-tert.butyl-2-éthoxyphényl)-cyclopropyl)-3-méthylpenta-2,4-diénoïque, l'acide(2E,4E)-(1RS,2RS)-5-[2-(3,5-di-tert.butyl-2-éthoxyphényl)-cyclopropyl]-3-méthyl-penta-2,4-diénoïque, et l'acide(2E,4E)-(1RS,2RS)-5-[2-(3,5-di-tert.butyl-2-butoxyphényl)-cyclopropyl]-3-méthyl-penta-2,4-diénoïque.

8. Composés de formule :

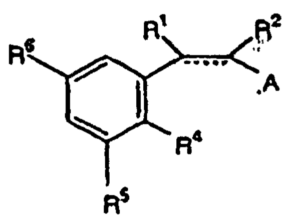

XVIII

dans laquelle A est un formyle; et $R^1$, $R^2$ et $R^4$ à $R^6$ sont comme défini dans la revendication 1 et la liaison en pointillés est facultative.

9. Préparations pharmaceutiques contenant un composé de formule I selon la revendication 1 ou des sels pharmaceutiquement acceptables d'un acide carboxylique de formule I et des matériaux véhiculaires pharmaceutiques usuels.

10. Utilisation de composés de formule I selon les revendications 1 à 7 ou de sels pharmaceutiquement acceptables d'un acide carboxylique de formule I dans la fabrication d'un médicament pour réduire ou éliminer des événements défavorables chez les patients recevant un traitement agoniste de rétinoïde.

11. Utilisation de composés de formule I selon les revendications 1 à 7 ou de sels pharmaceutiquement acceptables d'un acide carboxylique de formule I selon la revendication 10, dans laquelle le traitement agoniste de rétinoïde est un traitement pour l'acné, le psoriasis ou les maladies précancéreuses ou malignes.

12. Utilisation de composés de formule I selon les revendications 1 à 7 ou de sels pharmaceutiquement acceptables d'un acide carboxylique de formule I dans la fabrication d'un médicament pour le traitement de maladies immunes médiées par des cellules T auxiliaires de type 2 (Th2), telles que les maladies allergiques médiées par l'immunoglobuline E(IgE) ou pour le traitement de maladies médiées par des cytokines apparentées aux Th2, telles que IL-4 et IL-5.

**13.** Utilisation selon la revendication 12, dans laquelle la maladie est une dermatite atopique, une rhinite allergique ou de l'asthme bronchique allergique.

**14.** Utilisation de composés de formule I selon les revendications 1 à 7 ou de sels pharmaceutiquement acceptables d'un acide carboxylique de formule I dans la fabrication d'un médicament pour le traitement de l'ostéoporose.

**15.** Utilisation selon la revendication 14, dans laquelle l'ostéoporose est une ostéoporose postménopausique ou une ostéoporose sénile chez les sujets des deux sexes ou toutes formes d'ostéoporose secondaire.

**16.** Utilisation de composés de formule I selon les revendications 1 à 7 ou de sels pharmaceutiquement acceptables d'un acide carboxylique de formule I dans la fabrication d'un médicament pour le traitement des maladies prénéoplasiques ou néoplasiques.

**17.** Utilisation selon la revendication 16, dans laquelle les maladies prénéoplasiques sont des lésions précancéreuses de la peau et des muqueuses et les maladies néoplasiques sont des tumeurs solides de la peau, des muqueuses, de la tête et du cou, des poumons, de l'estomac, du colon, du sein, des ovaires, du col de l'utérus et de la prostate.

**18.** Utilisation selon l'une quelconque des revendications 10 à 17, dans laquelle l'antagoniste d'acide rétinoïque est un composé de formule I selon la revendication 1.

**19.** Utilisation selon la revendication 18, dans laquelle le composé de formule I est un composé de formule Ic :

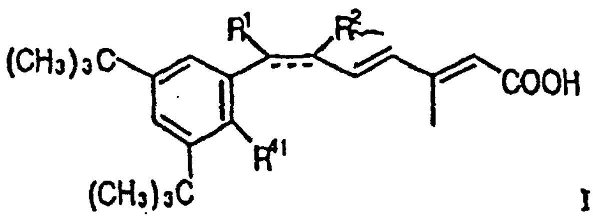

Ic

dans laquelle la liaison en pointillés est facultative; et, lorsque la liaison en pointillés est présente, $R^1$ est un méthyle et $R^2$ est l'hydrogène; et, lorsque la liaison en pointillés est absente, $R^1$ et $R^2$, pris conjointement, sont du méthylène pour former un cycle cyclopropyle substitué en cis et $R^{41}$ est un alcoxy en $C_{1-4}$.

**20.** Utilisation selon la revendication 19, dans laquelle le composé de formule Ic est l'acide (2E,4E)-(1RS,2RS)-5-[2-(3,5-di-tert.butyl-2-butoxyphényl)-cyclopropyl]-3-méthyl-penta-2,4-diénoïque.

# Figure 1

Comparison of the mean skin irritation scores evoked by
0.05% of Compound C alone and combined with 2.5% of Compound B

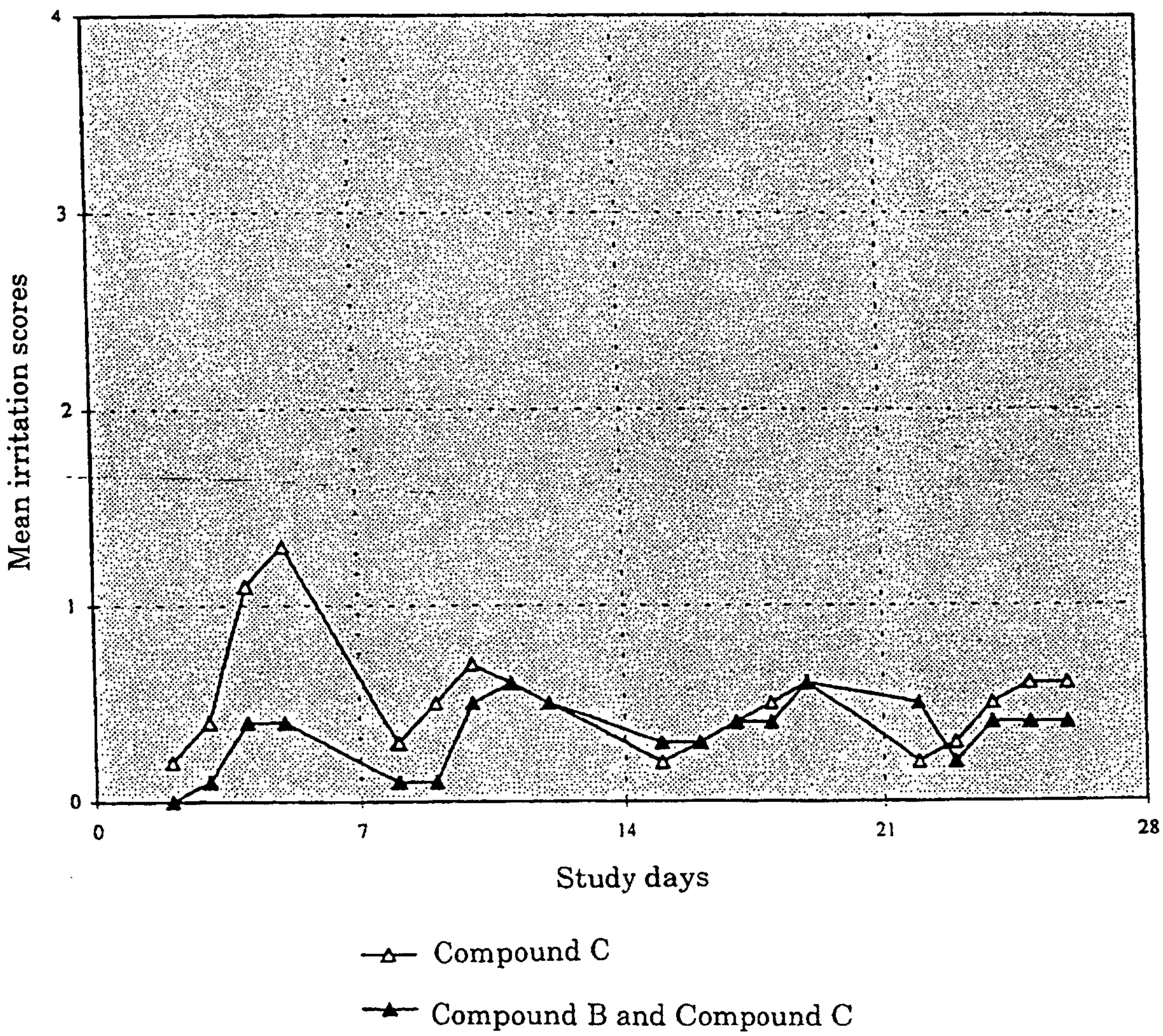

## Figure 2

**Comparison of the mean skin irritation scores evoked by 0.1% of Compound C alone and combined with 2.5% of Compound B**

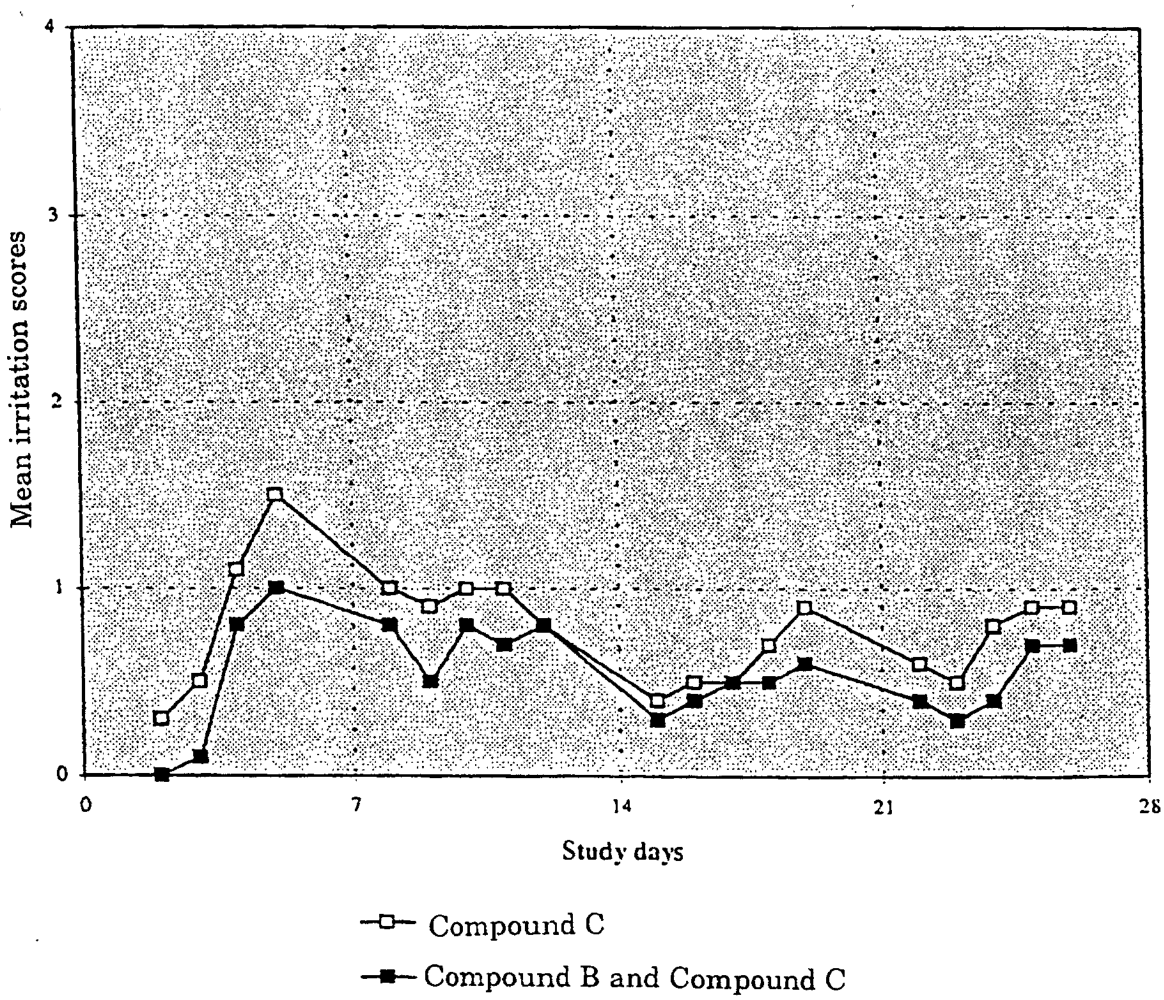

**Figure 3:**

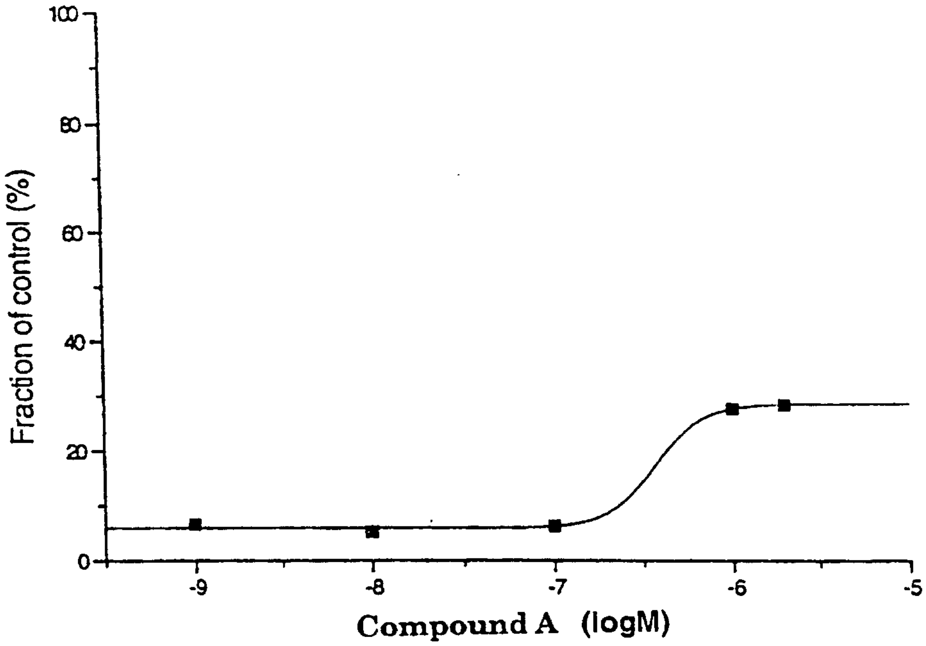


**Figure 4:**

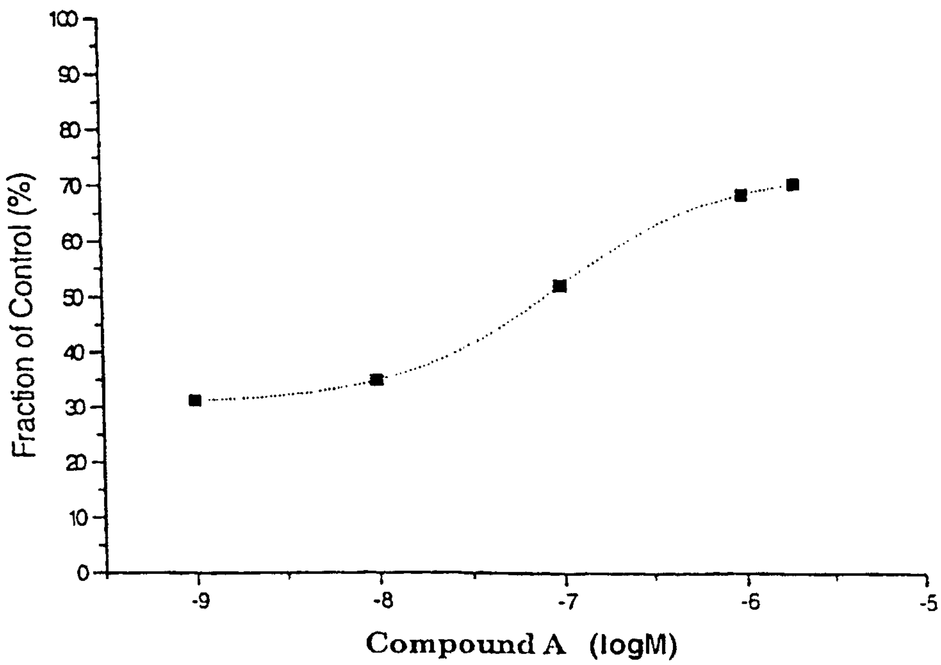

Figure 5:

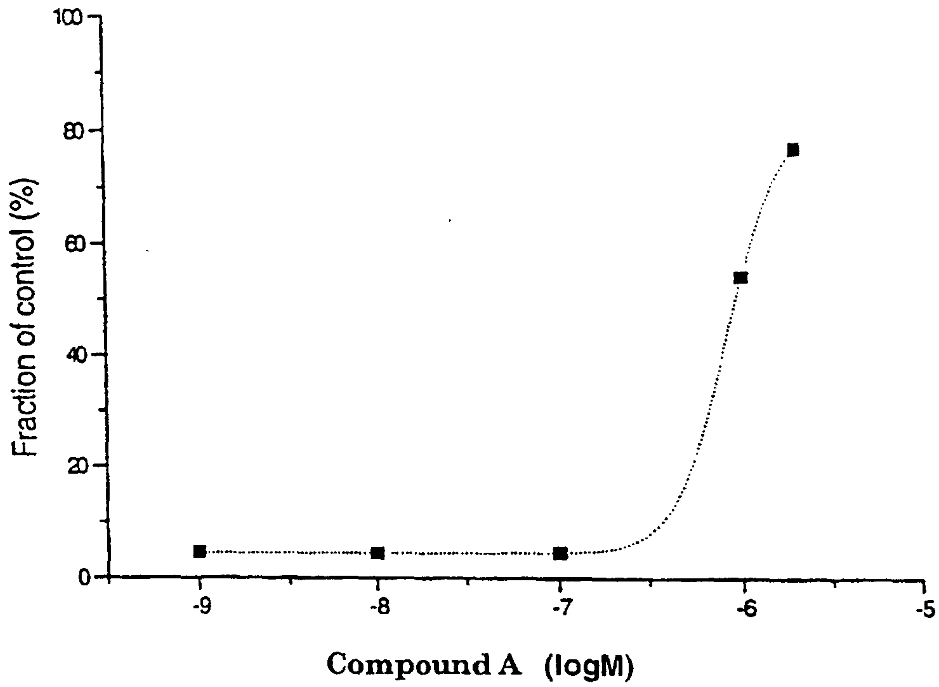


Figure 6:

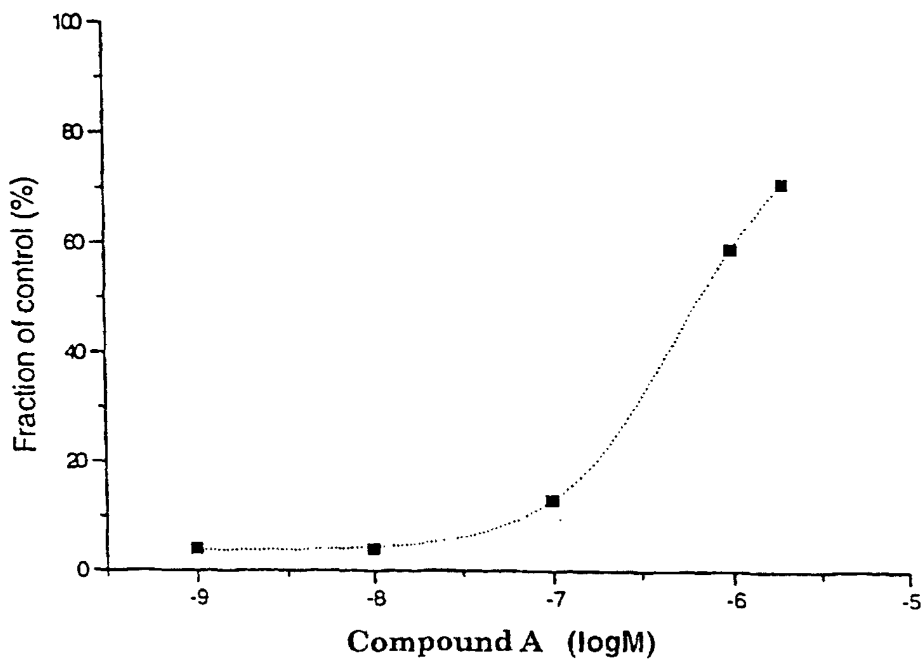

**Figure 7:**

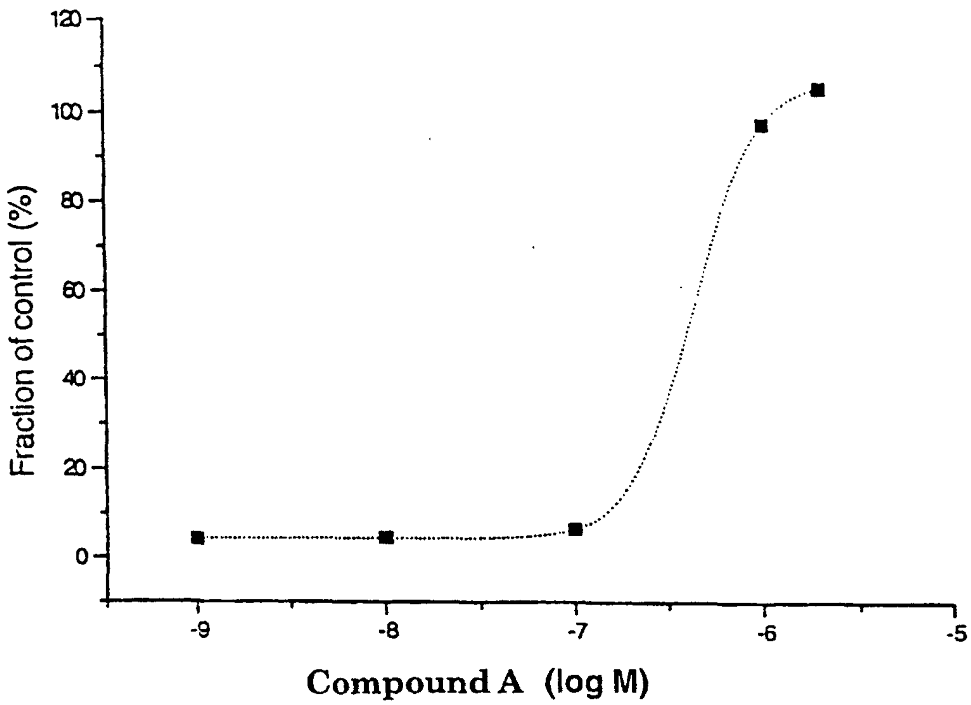

120
100
80
60
40
20
0
Fraction of control (%)
-9
-8
-7
-6
-5
Compound A (log M)